(19) Europäisches Patentamt
European Patent Office
Office européen des brevets

(11) **EP 3 120 134 B1**

(12) **FASCICULE DE BREVET EUROPEEN**

(45) Date de publication et mention de la délivrance du brevet:
**31.01.2018 Bulletin 2018/05**

(21) Numéro de dépôt: **15709113.3**

(22) Date de dépôt: **18.02.2015**

(51) Int Cl.:
*G01N 21/21* (2006.01)    *A61B 1/07* (2006.01)

(86) Numéro de dépôt international:
**PCT/EP2015/053437**

(87) Numéro de publication internationale:
**WO 2015/139907 (24.09.2015 Gazette 2015/38)**

(54) **DISPOSITIF ET MÉTHODE DE CARACTÉRISATION POLARIMÉTRIQUE DÉPORTÉE**

VORRICHTUNG UND VERFAHREN ZUR POLARIMETRISCHEN FERNBESTIMMUNG

DEVICE AND METHOD FOR REMOTE POLARIMETRIC CHARACTERIZATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.03.2014 FR 1452244**

(43) Date de publication de la demande:
**25.01.2017 Bulletin 2017/04**

(73) Titulaires:
• **Centre National de la Recherche Scientifique (C.N.R.S.)**
**75794 Paris Cedex 16 (FR)**
• **Ecole Polytechnique**
**91128 Palaiseau Cedex (FR)**

(72) Inventeurs:
• **PAGNOUX, Dominique**
**F-87100 Limoges (FR)**
• **VIZET, Jérémy**
**F-91300 Massy (FR)**
• **MANHAS, Sandeep**
**F-87000 Limoges (FR)**
• **VANEL, Jean-Charles**
**F-75020 Paris (FR)**
• **DEBY, Stanislas**
**F-92100 Boulogne Billancourt (FR)**
• **DE MARTINO, Antonello**
**décédé (FR)**

(74) Mandataire: **Osha Liang**
**2, rue de la Paix**
**75002 Paris (FR)**

(56) Documents cités:
• **JI QI ET AL: "Narrow band 3 x 3 Mueller polarimetric endoscopy", BIOMEDICAL OPTICS EXPRESS, vol. 4, no. 11, 2013, pages 2433-2449, XP055152270, ISSN: 2156-7085, DOI: 10.1364/BOE.4.002433 cité dans la demande**
• **JIAO S ET AL: "Cancellation of the polarization distortions in fiber-based polarization-sensitive Mueller-matrix optical coherence tomography", PROCEEDINGS OF SPIE, vol. 4956, 2003, pages 282-289, XP055152277, ISSN: 0277-786X, DOI: 10.1117/12.479033**

## Description

### Domaine technique

La présente description concerne une méthode et un dispositif de caractérisation polarimétrique déportée par fibre optique et s'applique notamment à l'endoscopie pour la caractérisation polarimétrique in vivo de tissus biologiques.

### Etat de l'art

Il est connu de décrire l'état de polarisation d'une onde électromagnétique (dont notamment la lumière visible) par un ensemble de quatre valeurs appelés paramètres de Stokes, souvent notés sous forme d'un vecteur, le vecteur de Stokes $\vec{S}$ :

$$\vec{S} = \begin{pmatrix} I_x + I_y \\ I_x - I_y \\ I_{45°} - I_{-45°} \\ I_G - I_D \end{pmatrix} = \begin{pmatrix} I \\ Q \\ U \\ V \end{pmatrix} = \begin{pmatrix} S_0 \\ S_1 \\ S_2 \\ S_3 \end{pmatrix} \qquad (1)$$

Le vecteur de Stokes comprend quatre composantes généralement notées $I$, $Q$, $U$, $V$ et qui décrivent respectivement l'intensité totale $I$ du faisceau ($I = I_x + I_y$), les différences entre les composantes horizontale et verticale du champ électrique ($I_x - I_y$), à $\pm$ 45° ($I_{45°} - I_{-45°}$) et circulaires gauche et droite ($I_G - I_D$). Ils permettent de décrire de façon complète la lumière non polarisée, partiellement polarisée et totalement polarisée.

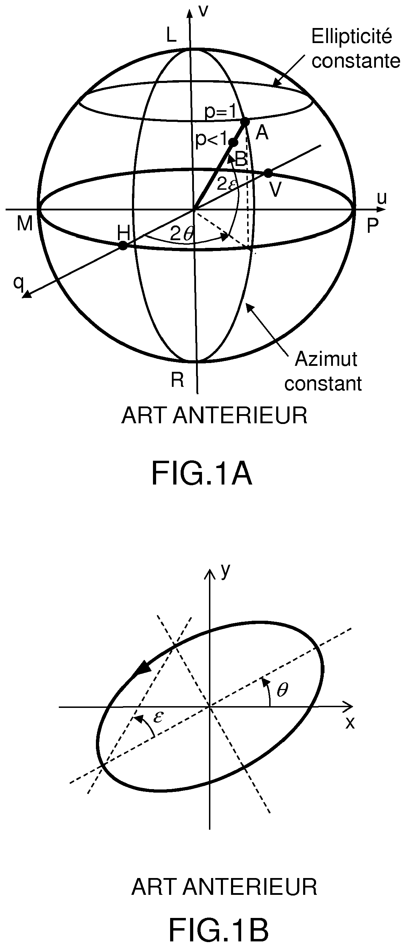

La sphère de Poincaré est une représentation graphique de la polarisation de la lumière, dont un exemple est illustré sur la figure 1A. Un vecteur de Stokes générique est représenté par ses coordonnées réduites $q=Q/I$, $u=U/I$ et $v=V/I$ qui définissent l'état de polarisation indépendamment de l'intensité globale. Les états totalement polarisés se trouvent sur la surface de la sphère, de rayon unité (par exemple le point référencé A sur la figure 1A). Les états partiellement polarisés, dont le degré de polarisation p est défini par:

$$p = q^2 + u^2 + v^2 \qquad (2)$$

se situent à l'intérieur de la sphère, à une distance $p<1$ du centre (par exemple le point référencé B sur la figure 1A). Les coordonnées polaires $\varepsilon$ et $\theta$ définissent respectivement l'ellipticité et l'azimut du grand axe d'une polarisation généralement elliptique telle que représentée sur la figure 1B.

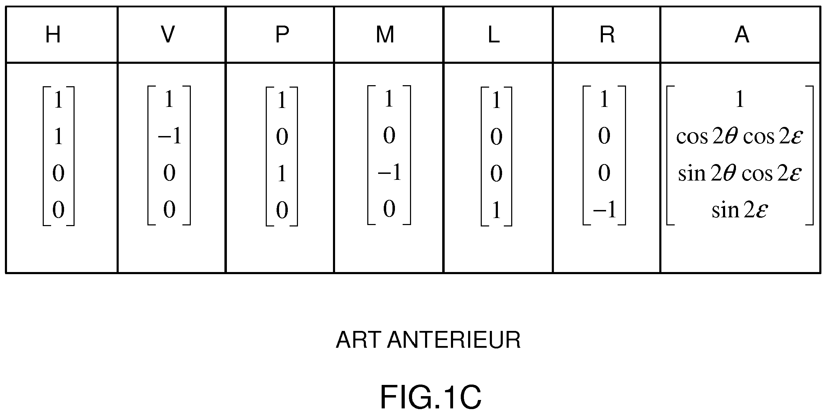

La figure 1C montre ainsi des exemples de vecteurs de Stokes d'ondes polarisées, de polarisations données, respectivement des vecteurs de Stokes correspondant à une polarisation linéaire horizontale (H), une polarisation linéaire verticale (V), des polarisations linéaires orientées à $\pm$45° (P et M), des polarisations circulaires droite (R) et gauche (L) et une polarisation elliptique (A), chacun des états de polarisation correspondant étant par ailleurs représenté par un point sur la sphère de Poincaré représentée sur la figure 1A.

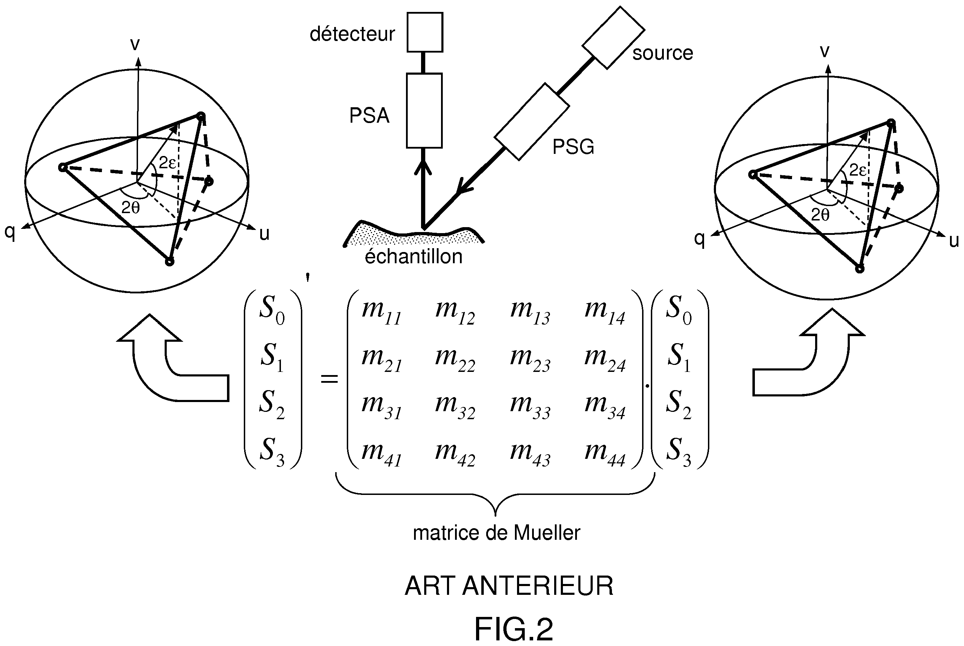

La matrice de Mueller d'un échantillon est un ensemble de 16 données qui déterminent complètement la réponse polarimétrique de cet échantillon et constituent un moyen de caractérisation structurelle de celui-ci. La technique permettant de mesurer cette matrice, dite "polarimétrie de Mueller", consiste à éclairer l'échantillon avec au moins quatre états de polarisation différents et à analyser les états de polarisation renvoyés, comme cela est illustré sur la figure 2. Plus précisément, une source d'émission lumineuse, par exemple un laser ou une diode laser, permet l'envoi d'un faisceau lumineux dans un dispositif appelé "générateur d'états de polarisation" ou PSG (selon l'abréviation de l'expression anglo-saxonne « Polarization State Generator ») pour générer quatre états de polarisation lumineux donnés et connus ("états de polarisation sonde"), chacun défini par un vecteur de Stokes tel que décrit précédemment. Le PSG est constitué par exemple d'une succession de polariseurs et de lames retard dont on peut changer les caractéristiques par exemple par un contrôle électrique. Ces lames peuvent par exemple être constituées de lames de cristaux liquides orientés par l'application d'une tension électrique de commande. Pour chacun des quatre états de polarisation sortant du PSG, on analyse l'état de polarisation de la lumière renvoyée par l'échantillon à l'aide d'un "analyseur d'états de polarisation" ou PSA (selon l'abréviation de l'expression anglo-saxonne « Polarization State Analyser »). Le PSA permet de mesurer l'intensité de l'onde lumineuse renvoyée à travers quatre filtres en polarisation qui peuvent être identiques à ceux qui ont permis de générer les états de polarisation sonde. Par exemple, le PSA peut être « l'image miroir » du PSG, avec les mêmes composants, mais traversés dans l'ordre inverse. Le PSA peut également être différent du PSG, mais dans tous les cas, il doit réaliser au moins quatre filtres en polarisation différents pour déterminer complètement le vecteur de Stokes de la lumière émergeant de l'échantillon. Finalement, on réalise ainsi au moins 16 mesures en intensité qui permettent de construire la matrice de Mueller de l'échantillon.

Différentes études ont montré comment l'analyse des coefficients de la matrice de Mueller permet de remonter aux informations polarimétriques concernant l'échantillon caractérisé (biréfringences linéaire et circulaire, diatténuation (ou dichroïsme) linéaire et circulaire, taux de dépolarisation). Par exemple, Lu et Chipman (S. Lu et al. « Interprétation of Mueller matrices based on polar décomposition », J. Opt.Soc. Am. A 13, 1106 - 1113 (1996)) ont démontré qu'il est possible de décomposer une matrice de Mueller non dégénérée en un produit de matrices, chacune étant caractéristique d'un effet optique spécifique, à savoir la *dépolarisation* (réduction du degré de polarisation tel que défini dans l'équation (2) ci-dessus lors de l'interaction avec l'échantillon), le *retard linéaire ou circulaire* (ou retard de phase introduit entre deux états de polarisation linéaire orthogonaux ou circulaires opposés) et la *diatténuation linéaire ou circulaire* (ou différence de transmission introduite entre deux états de polarisation linéaire orthogonaux ou circulaires opposés).

Les informations polarimétriques permettent alors de déterminer sur des échantillons biologiques des informations sur la structure physico-chimique des matériaux analysés. Par exemple, la *dépolarisation,* qui représente l'effet le plus important dans les tissus épais (autres que ceux de l'oeil), est due principalement à la diffusion multiple de la lumière sur des objets tels que les fibres ou nodules de collagène, les organelles intracellulaires, les noyaux, etc. Le *retard linéaire* s'observe sur des tissus minces (lames histologiques) ou épais en présence de protéines fibrillaires, telles que le collagène I, si ces fibres présentent une orientation préférentielle. La *diatténuation* est généralement négligeable, sauf dans le cas de tissus observés sous incidence rasante, où la traversée de l'interface peut créer une diatténuation significative, la polarisation dans le plan d'incidence étant mieux transmise que celle perpendiculaire à ce plan.

Ainsi, des études ex-vivo d'échantillons de colons (voir A. Pierangelo et al. Opt. Express 19, 1582-1593 (2011)) ont montré que ces échantillons se comportent comme des dépolariseurs purs, et que la dépolarisation fournit des contrastes utiles pour la détection de tumeurs à un stade précoce (elles dépolarisent moins que le tissu sain environnant), pour l'évaluation du degré de pénétration de tumeurs plus avancées ou encore pour la détection de tumeurs résiduelles après radiochimiothérapie (A. Pierangelo et al. J. Biomed. Opt.18, 046014 (2013)). Dans le cas de l'analyse des tissus du col utérin (A. Pierangelo et al; Opt. Express 21, 14120-30 (2013)), on observe à la fois de la dépolarisation et du retard, ce dernier étant présent uniquement dans les zones saines, dont il constitue donc un marqueur performant. La dépolarisation, elle, permet de distinguer les zones saines de celles présentant des lésions précancéreuses (dysplasies). La demande de brevet publiée WO 2007003840 montre comment la polarimétrie de Mueller peut être ajoutée en complément d'un *colposcope,* c'est à dire un microscope binoculaire à grande distance de travail destiné à l'examen détaillé du col utérin in vivo.

Pour l'analyse d'objets biologiques *in vivo,* ou de façon plus générale tout objet difficile d'accès, il y a un intérêt évident à la réalisation d'une caractérisation polarimétrique déportée, permettant de déporter de l'objet à analyser les ensembles source/PSG d'une part et PSA/détection/analyse d'autre part. Une telle caractérisation déportée peut se faire au moyen d'un guide de lumière comme une fibre optique par exemple. Dans ce cas, on connait les états de polarisation envoyés par le PSG dans la fibre optique en direction de l'objet, et on peut analyser avec le PSA les états de polarisation de la lumière en provenance de l'objet, après qu'ils aient traversé la fibre optique en retour. Mais cette fibre optique induit des perturbations des états de polarisation de la lumière qui la traverse, autant à l'aller (trajet source-objet d'intérêt) qu'au retour (trajet objet- système de détection et d'analyse). Ces perturbations, imprévisibles, incontrôlables, sont fortement dépendantes du conditionnement de la fibre optique (courbures, torsions...) et de l'environnement (température,...). Elles empêchent de connaître les états de polarisation réellement incidents sur l'objet, et d'avoir accès aux états de polarisation que cet objet renvoie dans la fibre optique pour les analyser. Dans ces conditions, la caractérisation de l'objet par polarimétrie de Mueller n'est plus possible.

Des solutions ont été proposées récemment pour tenter de s'affranchir des perturbations induites par la fibre optique, ou plus généralement par le guide d'onde utilisé pour déporter l'objet à analyser, afin d'accéder à des informations polarimétriques d'un échantillon.

Dans la demande de brevet publiée FR 2941047, une onde polarisée linéairement est envoyée à travers un dispositif capable d'engendrer un grand nombre d'états de polarisation bien répartis sur la sphère de Poincaré, ces états de polarisation étant envoyés à travers un guide d'onde, puis réfléchis sur l'objet, et la polarisation de l'onde réfléchie étant analysée après avoir traversé le guide d'onde en retour. Un rotateur de Faraday est positionné du côté distal, c'est-à-dire du côté de l'extrémité du guide d'onde à laquelle se trouve l'objet à analyser, le rotateur de Faraday permettant une rotation de 45° de la polarisation. Ce rotateur de Faraday a pour effet de compenser, pour chaque état de polarisation envoyé sur l'objet, le retard introduit par la fibre. Pour chacun de ces états de polarisation sonde, on mesure la fraction $F$ de l'intensité détectée en retour, portée par la polarisation parallèle à la polarisation linéaire envoyée. De l'ensemble des fractions $F$ mesurées, dépendantes des états de polarisation sonde, on détermine la valeur minimale $F_{min}$ et la valeur maximale $F_{max}$ à partir desquelles sont déduits le taux de dépolarisation et le retard de phase introduits par l'objet. Cependant, on ne peut pas avoir accès grâce à cette technique à la diatténuation ou au dichroïsme circulaire de l'échantillon.

Plus récemment, un dispositif à de mesure polarimétrique à travers une fibre monomode a été décrit (voir par exemple la demande de brevet FR 2977033 d'Alouni et al.) qui permet de détecter si l'orthogonalité de deux polarisations incidentes sur l'objet d'analyse a été brisée, ce qui peut être dû à la dépolarisation ou la diatténuation due à l'objet, mais sans pouvoir faire de distinction entre ces deux effets. En outre, le retard éventuellement introduit par l'objet n'est pas mesurable par ce procédé.

L'article de Wood *et al.* (T.C. Wood et al. « Polarization response measurement and simulation of rigid endoscopes », Biomedical Optics express 463, Vol. 1, N°2 (2010)) met en évidence et caractérise dans des endoscopes rigides commerciaux (autrement appelés laparoscopes) des effets de biréfringence notamment, qui sont attribués à une fenêtre d'entrée en saphir. L'article suggère de remplacer le saphir par un matériau non biréfringent et compatible avec les contraintes liées à la stérilisation pour limiter ces effets de biréfringence et permettre des caractérisations polarimétriques complètes déportées sur des échantillons in vivo. Il peut néanmoins subsister des effets de biréfringence résiduels qui peuvent s'avérer gênants, surtout s'ils varient dans le temps ou avec la position de l'instrument.

Qi *et al.* décrivent également un laparoscope équipé d'un polariseur linéaire à l'extrémité distale et une roue à polariseurs linéaires à différentes orientations côté proximal (Qi et al., « Narrow band 3X3 Mueller polarimetric endoscopy », Biomedical Optics Express, vol 4 n°11, (2013)). Cet appareil permet l'acquisition de matrices de Mueller partielles, limitées aux trois premières lignes et trois premières colonnes, moyennant la rotation de l'instrument autour de son axe pour faire varier l'orientation du polariseur à la détection. Cette approche présente deux limitations : d'une part on n'a pas accès aux retards et diatténuation circulaires, et d'autre part la rotation de l'instrument autour de son axe est réellement peu pratique en conditions réelles d'examen, en raison, notamment, de la nécessité de réaliser cette rotation autour de l'axe de l'endoscope avec une excellente précision, pour éviter que l'image se déplace dans le champ entre deux acquisitions. Même si cette condition est remplie, on peut craindre que des déformations « non rigides » des organes examinés in vivo se produisent au cours de la rotation, ce qui peut disqualifier la méthode dans bon nombre de situations.

La présente invention propose une méthode et un système de caractérisation déportée qui permettent d'accéder au moyen d'une fibre optique souple, à une caractérisation de la matrice de Mueller complète d'un échantillon. Il est ainsi possible d'avoir accès simultanément à toutes les informations polarimétriques de l'échantillon, y compris les diatténuations et retards linéaire et circulaire. Cette caractérisation complète de la matrice de Mueller présente de nombreux avantages pour l'analyse des échantillons biologiques notamment. En effet, même si dans la plupart des cas les tissus présentent des effets intrinsèques essentiellement linéaires, on peut observer simultanément, sous incidence rasante (ce qui peut être fréquent en endoscopie), une diatténuation significative à la traversée de la surface du tissu, qui peut donner lieu à une diatténuation circulaire si par ailleurs le tissu présente de la biréfringence linéaire.

RESUME

Selon un premier aspect, la présente description concerne un dispositif de caractérisation polarimétrique déportée d'un échantillon comprenant :

- une source d'émission d'au moins une onde lumineuse incidente à au moins une première longueur d'onde;
- une fibre optique monomode dans laquelle l'onde lumineuse incidente est destinée à se propager;
- un générateur d'états de polarisation agencé du côté proximal de la fibre optique et permettant la génération d'un nombre donné d'états de polarisation de l'onde lumineuse incidente, dits états sonde;
- un réflecteur destiné à être agencé du côté distal de la fibre optique ;
- un analyseur d'états de polarisation agencé du côté proximal de la fibre optique et permettant, pour chaque état sonde de l'onde incidente, l'analyse de la polarisation de l'onde lumineuse obtenue après propagation de l'onde incidente dans la fibre optique, réflexion du côté distal de la fibre optique et propagation inverse dans la fibre optique;
- des moyens de traitement permettant de déterminer :

  ◦ à partir d'une première caractérisation polarimétrique de la fibre optique, obtenue par l'analyse pour chaque état sonde, de la polarisation d'au moins une onde réfléchie du côté distale de la fibre optique au moyen du réflecteur et propagée en sens inverse dans la fibre optique, une matrice de Mueller associée à la fibre optique à la première longueur d'onde ;
  ◦ à partir d'une deuxième caractérisation polarimétrique de l'ensemble comprenant la fibre optique et l'échantillon, obtenue par l'analyse pour chaque état sonde de la polarisation, d'une onde renvoyée du côté distal de la fibre par l'échantillon et propagée en sens inverse dans la fibre optique, une matrice de Mueller associée audit ensemble à la première longueur d'onde ;
  ◦ à partir des matrices de Mueller associées respectivement à la fibre optique et à l'ensemble comprenant la fibre optique et l'échantillon, la matrice de Mueller associée à l'échantillon à la première longueur d'onde.

L'arrangement original du dispositif de caractérisation polarimétrique permet d'avoir accès à l'ensemble des informations polarimétriques d'un échantillon grâce à une détermination complète de la matrice de Mueller de cet échantillon.

Selon un ou plusieurs exemples de réalisation, la source d'émission permet l'émission d'une onde à la première longueur d'onde et l'émission d'une onde à une deuxième longueur d'onde distincte de la première longueur d'onde.

Selon un ou plusieurs exemples de réalisation, le réflecteur est avantageusement un réflecteur spectral permettant la réflexion d'une onde se propageant dans la fibre optique à la deuxième longueur d'onde pour la caractérisation polarimétrique de la fibre optique à la deuxième longueur d'onde et le passage de l'onde à la première longueur d'onde pour la caractérisation polarimétrique de l'ensemble comprenant la fibre optique et l'échantillon à la première longueur d'onde. Les moyens de traitement permettent de déterminer :

⚬ à partir de la caractérisation polarimétrique de la fibre optique à la deuxième longueur d'onde, une matrice de Mueller associée à la fibre optique à la deuxième longueur d'onde;
⚬ à partir de la matrice de Mueller associée à la fibre optique à la deuxième longueur d'onde, une matrice de Mueller associée à la fibre optique à la première longueur d'onde.

Cette première variante basée sur une séparation chromatique des ondes lumineuses permet une détermination simultanée des matrices de Mueller de la fibre optique d'une part et de l'ensemble comprenant la fibre optique et l'échantillon. Par ailleurs, elle ne nécessite pas d'éléments optiques actifs du côté distal de la fibre optique.

Selon un ou plusieurs exemples de réalisation, les deux longueurs d'onde sont distinctes mais proches ; typiquement, l'écart entre les deux longueurs d'onde n'excède pas 100 nm.

Selon un ou plusieurs exemples de réalisation, lorsque le retard de phase généré par la fibre optique est supérieur à $2\pi$, la caractérisation de la fibre optique peut être faite au moyen de deux longueurs d'onde distinctes.

Ainsi, selon cet exemple, la source d'émission permet en outre l'émission d'une onde à une troisième longueur d'onde distincte des première et deuxième longueurs d'onde. Le réflecteur spectral permet la réflexion d'ondes se propageant dans la fibre optique aux deuxième et troisième longueurs d'onde pour la caractérisation polarimétrique de la fibre optique aux deuxième et troisième longueurs d'onde. Par ailleurs, les moyens de traitement permettent de déterminer :

⚬ à partir d'une caractérisation polarimétrique de la fibre optique à la deuxième longueur d'onde et à partir d'une caractérisation polarimétrique de la fibre optique à la troisième longueur d'onde, respectivement une matrice de Mueller associée à la fibre optique à la deuxième longueur d'onde et une matrice de Mueller associée à la fibre optique à la troisième longueur d'onde;
⚬ à partir des matrices de Mueller associées à la fibre optique aux deuxième et troisième longueurs d'onde, la matrice de Mueller associée à la fibre optique à la première longueur d'onde.

Là encore, les longueurs d'onde sont distinctes mais restent proches, l'écart entre les longueurs d'onde restant avantageusement inférieur à 100 nm.

Selon un ou plusieurs exemples de réalisation, le réflecteur est un réflecteur commutable entre une position réfléchissante et une position passante. Un tel réflecteur permet, dans la position réfléchissante, la réflexion d'une onde se propageant dans la fibre optique à la première longueur d'onde pour la caractérisation polarimétrique de la fibre optique et, dans la position passante, la réflexion de l'onde par l'échantillon pour la caractérisation polarimétrique de l'ensemble comprenant la fibre optique et l'échantillon.

Cette deuxième variante présente l'avantage de pouvoir procéder à la caractérisation de la fibre optique directement à la première longueur d'onde, c'est-à-dire à la longueur d'onde utilisée pour caractériser l'ensemble comprenant la fibre optique et l'échantillon. Ceci permet notamment une plus grande souplesse dans le choix de la fibre optique monomode utilisée.

Selon un ou plusieurs exemples de réalisation, et quelle que soit la variante mise en oeuvre, la fibre optique monomode est une fibre optique à maintien de polarisation, qui élimine tout effet chiral.

Selon un ou plusieurs exemples de réalisation, la fibre optique monomode comprend un premier tronçon d'une fibre optique monomode à maintien de polarisation et un deuxième tronçon de la même fibre optique monomode à maintien de polarisation, les tronçons étant de même longueur et raccordés entre eux de telle sorte que l'axe rapide du premier tronçon soit aligné avec l'axe lent du deuxième tronçon et inversement.

Cet exemple de fibre permet notamment de réduire le retard de phase introduit par la fibre tout en éliminant tout effet chiral facilitant ainsi sa caractérisation.

Selon un ou plusieurs exemples de réalisation, le dispositif selon le premier aspect comprend en outre du côté distal de la fibre optique, des moyens de focalisation d'une onde à la première longueur d'onde pour la caractérisation d'une zone ponctuelle de l'échantillon.

Selon un ou plusieurs exemples de réalisation, le dispositif selon le premier aspect comprend en outre du côté distal de la fibre optique, des moyens de balayage pour la caractérisation polarimétrique d'un ensemble de zones ponctuelles de l'échantillon.

Selon un deuxième aspect, la présente description concerne une ou des méthode(s) de caractérisation polarimétrique déportée d'un échantillon mises en oeuvre par le(s) dispositif(s) selon le premier aspect.

Ainsi, l'invention concerne une méthode de caractérisation polarimétrique déportée d'un échantillon comprenant :

- l'émission d'une onde lumineuse incidente à au moins une première longueur d'onde destinée à se propager dans une fibre optique monomode;
- la caractérisation polarimétrique de la fibre optique à la première longueur d'onde, comprenant :

  ∘ la génération d'un nombre donné d'états de polarisation de l'onde lumineuse incidente, appelés états sonde, au moyen d'un générateur d'états de polarisation agencé du côté proximal de la fibre optique ;
  ∘ l'analyse au moyen d'un analyseur d'états de polarisation agencé du côté proximal de la fibre optique, pour chaque état sonde de l'onde incidente, de la polarisation de l'onde lumineuse obtenue après propagation de l'onde incidente dans la fibre optique, réflexion au moyen d'un réflecteur agencé du côté distal de la fibre optique et propagation inverse dans la fibre optique;
  ∘ la détermination d'une matrice de Mueller associée à la fibre optique à la première longueur d'onde ;

- la caractérisation polarimétrique de l'ensemble comprenant la fibre optique et l'échantillon à la première longueur d'onde, comprenant :

  ∘ au moyen desdits générateur d'états de polarisation et analyseur d'états de polarisation, l'analyse pour chaque état sonde de la polarisation, d'une onde renvoyée du côté distal de la fibre par l'échantillon et propagée en sens inverse dans la fibre optique;
  ∘ la détermination d'une matrice de Mueller associée audit ensemble à la première longueur d'onde ;

- la détermination de la matrice de Mueller associée à l'échantillon à la première longueur d'onde à partir des matrices de Mueller associées respectivement à la fibre optique et à l'ensemble comprenant la fibre optique et l'échantillon.

Selon un ou plusieurs exemples de réalisation, la méthode comprend en outre l'émission d'une onde lumineuse à une deuxième longueur d'onde distincte de la première longueur d'onde. Selon cette variante, le réflecteur est un réflecteur spectral permettant la réflexion d'une onde se propageant dans la fibre optique à la deuxième longueur d'onde pour la caractérisation polarimétrique de la fibre optique à la deuxième longueur d'onde et le passage de l'onde à la première longueur d'onde pour la caractérisation polarimétrique de l'ensemble comprenant la fibre optique et l'échantillon à la première longueur d'onde. Par ailleurs, la détermination de la matrice de Mueller associée à la fibre optique à la première longueur d'onde comprend :

  ∘ à partir de la caractérisation polarimétrique de la fibre optique à la deuxième longueur d'onde, une matrice de Mueller associée à la fibre optique à la deuxième longueur d'onde;
  ∘ à partir de la matrice de Mueller associée à la fibre optique à la deuxième longueur d'onde, une matrice de Mueller associée à la fibre optique à la première longueur d'onde.

Selon un ou plusieurs exemples de réalisation, la méthode comprend l'émission d'une onde à une troisième longueur d'onde distincte des première et deuxième longueurs d'onde, et le réflecteur spectral permet la réflexion d'ondes se propageant dans la fibre optique aux deuxième et troisième longueurs d'onde pour la caractérisation polarimétrique de la fibre optique aux deuxième et troisième longueurs d'onde; la détermination de la matrice de Mueller associée à la fibre optique à la première longueur d'onde comprend :

  ∘ à partir d'une caractérisation polarimétrique de la fibre optique à la deuxième longueur d'onde et à partir d'une caractérisation polarimétrique de la fibre optique à la troisième longueur d'onde, respectivement une matrice de Mueller associée à la fibre optique à la deuxième longueur d'onde et une matrice de Mueller associée à la fibre optique à la troisième longueur d'onde;
  ∘ à partir des matrices de Mueller associées à la fibre optique aux deuxième longueur d'onde et troisième longueur d'onde, la matrice de Mueller associée à la fibre optique à la première longueur d'onde.

Selon un ou plusieurs exemples de réalisation, le réflecteur est un réflecteur commutable entre une position réfléchissante et une position passante, permettant, dans la position réfléchissante, la réflexion d'une onde se propageant dans la fibre optique à la première longueur d'onde pour la caractérisation polarimétrique de la fibre optique et, dans la position passante, la réflexion de l'onde par l'échantillon pour la caractérisation polarimétrique de l'ensemble comprenant la fibre optique et l'échantillon.

Selon un ou plusieurs exemples de réalisation, la méthode selon l'une des variantes décrites précédemment comprend en outre, du côté distal de la fibre optique, la focalisation d'une onde lumineuse à la première longueur d'onde aux moyens de focalisation pour la caractérisation d'une zone ponctuelle de l'échantillon.

La méthode peut également comprendre, du côté distal de la fibre optique, le balayage par des moyens de balayage de l'onde lumineuse focalisée pour la caractérisation polarimétrique d'un ensemble de zones ponctuelles de l'échantillon.

BREVE DESCRIPTION DES DESSINS

D'autres avantages et caractéristiques apparaîtront à la lecture de la description, illustrée par les figures suivantes :

- Figures 1A et 1B, une représentation de la sphère de Poincaré et d'un état de polarisation elliptique (déjà décrites) ;
- Figure 1C, un tableau montrant les composantes de vecteurs de Stokes de différents états de polarisation (déjà décrit) ;
- Figure 2, un schéma illustrant un montage expérimental pour une caractérisation par polarimétrie de Mueller selon l'art antérieur (déjà décrit) ;
- Figure 3, un schéma illustrant un dispositif de caractérisation polarimétrique selon la présente description, selon un premier exemple ;
- Figure 4, un schéma illustrant un dispositif de caractérisation polarimétrique selon la présente description, selon un deuxième exemple ;
- Figure 5, un schéma montrant selon une vue partielle, une variante d'un dispositif de caractérisation polarimétrique selon la présente description ;
- Figure 6, un schéma illustrant un montage expérimental utilisé pour la validation expérimentale d'un exemple de méthode de caractérisation selon la présente description ;
- Figures 7 et 8, des courbes expérimentales obtenues avec le schéma montré sur la figure 6 et comparées avec des valeurs théoriques attendues ;
- Figure 9A, un schéma partiel du schéma de la figure 6, montrant un échantillon utilisé pour la validation d'un exemple de méthode de caractérisation selon la présente description ;
- Figure 9B, des courbes expérimentales obtenues avec l'échantillon montré sur la figure 9A et comparées avec des valeurs théoriques attendues ;
- Figure 10A, un schéma partiel du schéma de la figure 6, montrant un échantillon utilisé pour la validation d'un exemple de méthode de caractérisation selon la présente description ;
- Figure 10B, des courbes expérimentales obtenues avec un échantillon du type de celui montré sur la figure 10A et comparées avec des valeurs théoriques attendues ;
- Figure 11A, un schéma partiel du schéma de la figure 6, montrant un autre échantillon utilisé pour la validation d'un exemple de méthode de caractérisation selon la présente description ;
- Figure 11B, des courbes expérimentales obtenues avec un échantillon du type de celui montré sur la figure 11A et comparées avec des valeurs théoriques attendues.

DESCRIPTION DETAILLEE

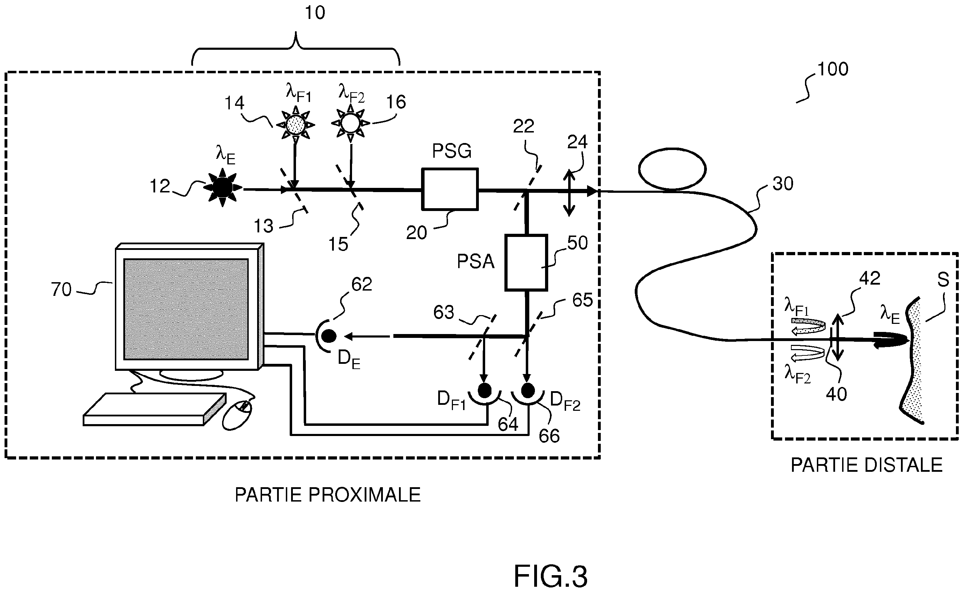

La figure 3 représente un dispositif de caractérisation polarimétrique 100 selon un premier exemple, pour la mise en oeuvre d'une méthode de caractérisation polarimétrique selon un premier aspect de la présente description.

Le dispositif de caractérisation polarimétrique 100 comprend de façon générale une source d'émission 10 d'au moins une onde lumineuse à au moins une première longueur d'onde $\lambda_E$ et une fibre optique monomode 30 dans laquelle l'onde lumineuse est destinée à se propager pour une caractérisation déportée d'un échantillon S. Une fibre optique monomode présente notamment l'avantage par rapport à une fibre multimode de ne pas dépolariser la lumière incidente même si elle peut être amenée selon sa nature et les conditions expérimentales à modifier la polarisation. L'échantillon à analyser ou « objet d'analyse » S est situé, par rapport à la source d'émission 10, à l'autre extrémité de la fibre optique 30.

Dans la suite de la description, on appellera partie proximale du dispositif de caractérisation polarimétrique toute la partie du dispositif située du côté de la fibre optique où se trouve la source d'émission et partie distale du dispositif toute la partie du dispositif située à l'autre extrémité de la fibre optique, c'est-à-dire où se trouve l'échantillon.

Le dispositif 100 comprend en outre un générateur d'états de polarisation PSG agencé du côté proximal de la fibre optique et permettant la génération d'un nombre donné d'états de polarisation de l'onde lumineuse (états de polarisation sonde) et un analyseur d'états de polarisation PSA agencé du côté proximal de la fibre optique et permettant l'analyse des états de polarisation de l'onde lumineuse après réflexion du côté distal de la fibre optique et propagation inverse dans la fibre optique.

Dans l'exemple de la figure 3, la source 10 permet l'émission d'une onde à une première longueur d'onde $\lambda_E$ et l'émission d'au moins une onde à une deuxième longueur d'onde $\lambda_{F1}$ distincte de la première longueur d'onde $\lambda_E$. Selon une variante qui sera décrite en détails par la suite, la source 10 permet l'émission de deux ondes respectivement aux longueurs d'onde $\lambda_{F1}$ et $\lambda_{F2}$, distinctes entre elles et distinctes de $\lambda_E$. La source 10 comprend par exemple un ensemble de sources monochromatiques, par exemple des diodes laser, respectivement notées 12, 14, 16 sur la figure 3, chacune émettant à l'une des longueurs d'onde distinctes. Alternativement, la source 10 peut être constituée d'une source unique émettant à l'ensemble des longueurs d'onde, par exemple une source de type "source de supercontinuum" engendré par élargissement spectral d'un faisceau laser au sein d'une fibre optique.

Avantageusement, les sources d'émission sont des sources continues. Alternativement, il peut s'agir de sources impulsionnelles de puissances crêtes suffisamment faibles pour ne pas générer dans la fibre optique d'effets optiques non linéaires. Une autre option consiste à utiliser une source continue modulée par un hacheur et mettre en oeuvre une détection synchrone sur tous les détecteurs, afin d'améliorer, éventuellement, le rapport signal à bruit par rapport à une source continue. Dans ce cas, la modulation est avantageusement effectuée à une fréquence bien supérieure à la fréquence de commutation des cristaux liquides des PSG et PSA, qui est au plus de l'ordre du kHz, ce qui ne pose pas de problème a priori, la détection synchrone étant typiquement mise en oeuvre jusqu'à des fréquences de 100 kHz avec des systèmes commerciaux.

Le dispositif de caractérisation polarimétrique comprend en outre dans l'exemple décrit sur la figure 3 un réflecteur 40 sélectif en longueurs d'onde, agencé du côté distal de la fibre optique, ainsi que des moyens de focalisation 42. Le réflecteur 40 permet de réfléchir les ondes à la longueur d'onde $\lambda_{F1}$, ou aux longueurs d'onde $\lambda_{F1}$ et $\lambda_{F2}$, tout en laissant passer les ondes à la longueur d'onde $\lambda_E$. Le réflecteur 40 est par exemple un filtre spectral placé en sortie de la fibre optique 30 ; ce filtre peut être par exemple un filtre passe haut coupant entre $\lambda_{F2}$ et $\lambda_E$, si l'on choisit $\lambda_{F1} < \lambda_{F2} < \lambda_E$. Ce filtre peut aussi, par exemple, être un filtre passe bande autour de $\lambda_E$ si l'on choisit $\lambda_{F1} < \lambda_E < \lambda_{F2}$ ou passe bas si l'on choisit $\lambda_E < \lambda_{F1} < \lambda_2$.

Le dispositif de caractérisation polarimétrique 100 comprend par ailleurs une lame séparatrice 22 et des éléments séparateurs de longueurs d'onde 65, 63, par exemple des filtres spectraux, agencés après l'analyseur d'états de polarisation PSA, et permettant de séparer en fonction de la longueur d'onde, les ondes rétroréfléchies ou rétrodiffusées du côté distal de la fibre et propagées en retour dans la fibre. Les ondes lumineuses ainsi séparées sont envoyées vers des photodétecteurs respectivement notés $D_E$, $D_{F1}$, $D_{F2}$ sur la figure 3, par exemple des photodiodes, sensibles respectivement aux longueurs d'onde $\lambda_E$, $\lambda_{F1}$ et $\lambda_{F2}$.

Le dispositif de caractérisation polarimétrique 100 comprend également des moyens de traitement 70 permettant à partir des signaux électroniques émis par les photodétecteurs de déterminer la matrice de Mueller de l'échantillon S, comme cela est décrit ci-dessous. Les moyens de traitement 70 assurent notamment la commande et la synchronisation des PSG et PSA, la collecte et le traitement des signaux émis par les photodétecteurs, la construction des matrices de Mueller.

Le principe général de la méthode de caractérisation polarimétrique selon la première variante mise en oeuvre par exemple au moyen d'un dispositif tel que décrit sur la figure 3 est basé sur des mesures simultanées faites à des longueurs d'onde différentes mais proches, typiquement séparées de moins de 100 nm, pour caractériser la fibre optique d'une part et l'ensemble fibre - objet d'autre part. Plus précisément, une ou deux longueurs d'onde peuvent être utilisées pour caractériser la fibre ($\lambda_{F1}$ et $\lambda_{F2}$), et une suffit pour l'ensemble fibre-objet d'intérêt ($\lambda_E$).

Ainsi, des ondes lumineuses, par exemple des ondes monochromatiques ou quasi monochromatiques (typiquement de largeurs spectrales inférieures à 40 nm) sont envoyées dans la fibre optique monomode 30 au moyen d'une lentille d'injection 24. Après la traversée du réflecteur sélectif en longueurs d'onde 40, le faisceau à la longueur d'onde $\lambda_E$ est focalisé sur l'objet par les moyens de focalisation 42, par exemple une lentille ou tout autre élément optique capable de réaliser la fonction de focalisation. Une partie de la lumière renvoyée par cet objet, toujours à $\lambda_E$, retraverse l'élément optique de focalisation 42 et le filtre spectral 40 pour être réinjectée dans la fibre optique 30. Le ou les faisceau(x) aux longueurs d'onde différentes de $\lambda_E$, dans cet exemple les longueurs d'onde $\lambda_{F1}$ et $\lambda_{F2}$, sont quant à eux réfléchis par le filtre spectral 40 et réinjectés dans la fibre optique 30 sans être impactés par l'échantillon S. En retour, l'ensemble des faisceaux à $\lambda_{F1}$, $\lambda_{F2}$ et $\lambda_E$ sont déviés en direction du PSA, puis séparés à la sortie du PSA au moyen des éléments séparateurs 65, 63 vers les détecteurs 62, 64, 66. Dans le cas de l'utilisation d'une source unique à large spectre émettant les longueurs d'onde $\lambda_E$, $\lambda_{F1}$ et $\lambda_{F2}$, un filtre spectral à bande étroite (typiquement < 40 nm) peut avantageusement être placé devant chacun des détecteurs 62, 64 et 66 pour ne laisser passer qu'une bande spectrale étroite autour de $\lambda_E$, $\lambda_{F1}$ et $\lambda_{F2}$ respectivement.

On décrit dans un premier temps un exemple de mise en oeuvre de la méthode selon la présente description, dans lequel une deuxième longueur d'onde $\lambda_{F1}$ est utilisée pour caractériser la fibre optique monomode 30.

Selon cet exemple, il est procédé à la caractérisation polarimétrique de la fibre optique à la deuxième longueur d'onde $\lambda_{F1}$, puis à la détermination d'une matrice de Mueller associée à la fibre optique à la deuxième longueur d'onde $\lambda_{F1}$. Une matrice de Mueller ($\mathbf{M_F}$) associée à la fibre optique à la première longueur d'onde $\lambda_E$ est déduite de la matrice de Mueller associée à la fibre optique à la deuxième longueur d'onde $\lambda_{F1}$. Simultanément à la caractérisation de la fibre optique à la deuxième longueur d'onde $\lambda_{F1}$, une caractérisation polarimétrique de l'ensemble comprenant la fibre optique et l'échantillon est obtenue à la première longueur d'onde $\lambda_E$ par l'analyse des états de polarisation d'une onde rétroréfléchie et/ou rétrodiffusée par l'échantillon. Une matrice de Mueller ($\mathbf{M_T}$) associée audit ensemble à la première longueur d'onde $\lambda_E$ est obtenue à partir de cette caractérisation. Il est alors possible à partir des matrices de Mueller associées respectivement à la fibre optique ($\mathbf{M_F}$) et à l'ensemble comprenant la fibre optique et l'échantillon ($\mathbf{M_T}$), de déterminer la matrice de Mueller ($\mathbf{M_O}$) associée à l'échantillon à la longueur d'onde $\lambda_E$.

Il est à noter que la caractérisation polarimétrique de la fibre optique comprend de façon générale la caractérisation de la fibre optique et de l'ensemble des éléments compris entre l'extrémité de sortie de la fibre optique (extrémité distale) et le réflecteur 40. Dans certains cas, cette caractérisation pourra être assimilée à une caractérisation de la fibre optique seule, soit parce qu'il n'y a pas d'autres éléments, soit parce que ces éléments ne modifient pas la polarisation. Par ailleurs, les éléments optiques qui peuvent se trouver entre le réflecteur sélectif 40 et l'échantillon S sont choisis de nature à ne pas modifier la polarisation des ondes propagées. C'est le cas par exemple de lentilles, ou autres éléments optiques tels que des miroirs sélectifs en fréquence, ou plus généralement des lames constituées de matériaux optiquement isotropes et portant éventuellement des couches diélectriques ou métalliques, ces lames étant utilisées à des incidences très proches de la normale (tolérance typique de l'ordre de 5°).

La caractérisation polarimétrique à une longueur d'onde donnée d'un objet à analyser, en l'occurrence la fibre optique ou l'ensemble fibre optique/échantillon est faite de façon connue et décrite par exemple dans le brevet européen EP 1411333. L'onde lumineuse est envoyée sur le générateur d'états de polarisation PSG, qui peut être par exemple commandé électriquement, afin de définir les 4 états de polarisation sonde. Avantageusement, ces états de polarisation sont les plus indépendants possibles. Ils sont alors répartis sur la sphère de Poincaré selon un tétraèdre régulier. En pratique, il est possible de travailler avec un plus grand nombre d'états de polarisation sonde mais on montre que 4 états sonde sont le nombre minimum d'états sonde pour l'analyse polarimétrique. Pour générer les états de polarisation sonde, le PSG comprend par exemple et de façon connue un ensemble d'éléments dont un polariseur linéaire, une première cellule à cristaux liquides commandable électriquement, une lame quart d'onde et une deuxième cellule à cristaux liquides commandable électriquement. Les 4 vecteurs de Stokes correspondant aux 4 états de polarisation ainsi générés sont agencés selon 4 colonnes pour former une matrice de modulation 4x4 notée **W**. Après interaction avec l'échantillon, les états de polarisation renvoyés sont analysés au moyen de l'analyseur d'états de polarisation PSA, qui comprend des éléments identiques à ceux du PSG mais agencés dans le sens inverse par rapport à la direction de la lumière, de telle sorte que, par exemple, la lumière traverse d'abord la deuxième cellule à cristaux liquides, puis la lame quart d'onde, puis la première cellule à cristaux liquides et le polariseur linéaire. Les vecteurs de Stokes correspondant aux 4 états de polarisation analysés par le PSA sont agencés selon 4 lignes pour former une matrice d'analyse 4x4 notée **A**. Ainsi, pour chacun des 4 états de polarisation issus du PSG, on mesure au moyen d'un détecteur l'intensité lumineuse en sortie du PSA, selon chacun des états de polarisation analysés. On obtient une matrice **B** des 16 niveaux d'intensités lumineuses mesurés telle que :

$$\mathbf{B} = \mathbf{A.M.W} \qquad (4)$$

Où **M** est la matrice de Mueller de l'échantillon.

L'inversion des matrices connues **A** et **W** permet alors de déterminer la matrice de Mueller selon la formule :

$$\mathbf{M} = \mathbf{A^{-1}.B.W^{-1}} \qquad (5)$$

Avantageusement, une calibration peut être opérée pour corriger des imperfections et erreurs d'alignement des éléments constituant les PSG et PSA. En effet, les matrices de modulation et d'analyse **W** et **A** peuvent être différentes des valeurs calculées théoriquement en fonction des éléments constituant le générateur et analyseur des états de polarisation. Pour pratiquer cette calibration, on peut par exemple placer successivement à la place de l'échantillon 4 échantillons de calibration qui permettront d'obtenir respectivement 4 matrices d'intensités. A partir d'algorithmes de calibration décrits dans la littérature (voir par exemple le brevet EP 1411333 précité), on peut alors obtenir les matrices de modulation et d'analyse **W** et **A** réelles.

Dans l'exemple de caractérisation polarimétrique d'un échantillon cité plus haut, les PSG et PSA comportent des cristaux liquides (nématiques ou ferroélectriques). De nombreux autres systèmes peuvent être utilisés pour la mise en oeuvre de la méthode selon la présente description. Par exemple, le PSG peut contrôler la polarisation au moyen de cellules de Pockels (voir par exemple E. Compain et al. "Complete Mueller matrix measurement with a single high frequency modulation," Thin Solid Films 313-314, 47-52, 1998) ou au moyen d'un modulateur photoélastique (voir par exemple E. Compain et al., "Complete high-frequency measurement of Mueller matrices based on a new coupled-phase modulator," Rev. Sci. Instrum. 68, 2671-2680 ~1997). Ces systèmes permettent de coder les quatre états du PSG simultanément sur quatre fréquences différentes. Côté PSA, on peut envisager l'utilisation de systèmes diviseurs d'amplitude, comme le « DOAP » décrit par E. Compain et al. (voir par exemple le brevet US 6177995 B1) et qui utilise un prisme séparateur et quatre détecteurs en parallèle. Ce type de PSA peut être avantageusement couplé à un PSG à codage fréquentiel ; le signal de chacun des détecteurs peut ainsi être démodulé sur les quatre fréquences du PSG, l'ensemble des signaux démodulés fournissant ainsi les 16 mesures à partir desquelles on peut obtenir la matrice de Mueller. Un exemple de ce type d'instrument est décrit dans le brevet US 6175412 B1. D'autres PSG et PSA utilisent des polariseurs linéaires fixes et des retardateurs tournants (voir par exemple le brevet US 7298480 B2).

La mise en oeuvre de la méthode de caractérisation grâce au dispositif montré sur la figure 3 permet de mesurer dans un premier temps une matrice de Mueller $\mathbf{M_{m1}}(\lambda_{F1})$ de la fibre optique à la deuxième longueur d'onde $\lambda_{F1}$, telle que :

$$\mathbf{M_{m1}}(\lambda_{F1}) = \mathbf{R}(-\theta_1). \, \mathbf{M_F^R}(\lambda_{F1}). \, \mathbf{M_F}(\lambda_{F1}). \, \mathbf{R}(\theta_1) \qquad (3)$$

Où $\mathbf{M_F}(\lambda_{F1})$ et $\mathbf{M_F^R}(\lambda_{F1})$ sont respectivement les matrices de Mueller de la fibre optique, à l'aller et au retour, et où $\mathbf{R}(\theta_1)$ et $\mathbf{R}(-\theta_1)$ sont respectivement des matrices de rotations d'angles $\theta_1$ et $-\theta_1$, $\theta_1$ étant l'angle inconnu a priori entre les axes neutres de la fibre à l'entrée de celle-ci et le repère du laboratoire dans lequel les vecteurs de Stokes sont définis. Dans l'hypothèse où la fibre se comporte comme un déphaseur pur, le produit $\mathbf{M_F^R}(\lambda_{F1}). \, \mathbf{M_F}(\lambda_{F1})$ correspond à la matrice d'un déphaseur linéaire pur représentant cette fibre sur un aller retour. Les angles $\theta_1$ et $-\theta_1$ sont donc déterminés de sorte que le produit $\mathbf{R}(-\theta_1)^{-1}. \, \mathbf{M_{m1}}(\lambda_{F1}). \, \mathbf{R}(\theta_1)^{-1} = \mathbf{M_F^R}(\lambda_{F1}). \, \mathbf{M_F}(\lambda_{F1})$ corresponde à la matrice d'un tel déphaseur linéaire pur. Alternativement, ces angles peuvent être connus à partir de la position en rotation de la fibre, dont les axes neutres auront été préalablement repérés. Si la fibre ne présente pas d'effets chiraux (diatténuation et retard *circulaires*), ce qui est le cas pour une fibre monomode standard si l'on prend bien soin de ne pas la « tordre », les matrices $\mathbf{M_F^R}(\lambda_{F1})$ et $\mathbf{M_F}(\lambda_{F1})$ sont celles de deux retardateurs linéaires identiques. On déduit facilement du produit $\mathbf{M_F^R}(\lambda_{F1}). \, \mathbf{M_F}(\lambda_{F1})$ mesuré directement chacune des matrices $\mathbf{M_F}(\lambda_{F1})$ et $\mathbf{M_F^R}(\lambda_{F1})$ de la fibre, respectivement aller et retour, à la première longueur d'onde $\lambda_E$. Or la matrice de Mueller $\mathbf{M_T}$ à la première longueur d'onde $\lambda_E$ de l'ensemble fibre optique et échantillon, qui est par ailleurs directement mesurée, est donnée par l'équation :

$$\mathbf{M_T}(\lambda_E) = \mathbf{M_F^R}(\lambda_E).\mathbf{M_O}. \, \mathbf{M_F}(\lambda_E) \qquad (4)$$

Où $\mathbf{M_O}$ est la matrice de Mueller recherchée de l'échantillon à la longueur d'onde $\lambda_E$.

Dans certains cas, il pourra par ailleurs être utile de déterminer l'angle $\theta_2$ définissant l'azimut de l'un des axes propres de la fibre à sa *sortie*, i.e. à son extrémité *distale*, par rapport au repère du laboratoire, afin de corriger la matrice $\mathbf{M_O}$ précédemment obtenue d'éventuels effets chiraux. Dans le cas de fibres à maintien de polarisation par exemple, cet azimut est lié à la fibre et peut être déterminé « mécaniquement ».

On peut ainsi déduire par inversion des matrices, la matrice de Mueller de l'échantillon :

$$\mathbf{M_O} = (\mathbf{M_F^R}(\lambda_E))^{-1}. \, \mathbf{M_T}(\lambda_E). \, (\mathbf{M_F}(\lambda_E))^{-1} \qquad (5)$$

La méthode décrite ci-dessus pourra bien fonctionner si les longueurs d'onde $\lambda_{F1}$ et $\lambda_E$ sont distinctes mais suffisamment proches, c'est-à-dire présentant une différence inférieure à 100 nm, avantageusement inférieure à 50 nm, et si la fibre se comporte bien comme un retardateur linéaire avec des axes neutres de directions bien identifiées, de telle sorte que la fibre optique induise un retard de phase $\delta\varphi_{F1}$ selon les axes neutres suffisamment faible (entre 0 et $2\pi$). Dans ce cas, il est possible de déduire de la matrice de la fibre optique à la deuxième longueur d'onde $\lambda_{F1}$, la matrice de la fibre optique à la première longueur d'onde $\lambda_E$ en déduisant le retard de phase $\delta\varphi_E$ à $\lambda_E$ à partir du retard de phase $\delta\varphi_{F1}$ à $\lambda_{F1}$ ($\delta\varphi_E = \delta\varphi_{F1} * \lambda_{F1} / \lambda_E$).

Selon une deuxième variante de la méthode de caractérisation polarimétrique pouvant être mise en oeuvre également avec un dispositif tel que celui représenté sur la figure 3, deux longueurs d'onde $\lambda_{F1}$, $\lambda_{F2}$ distinctes entre elles et distinctes de la première longueur d'onde $\lambda_E$ sont utilisées pour la caractérisation polarimétrique de la fibre optique.

Ainsi selon cette variante, la source d'émission permet en outre l'émission d'une onde à une troisième longueur d'onde $\lambda_{F2}$ distincte des première et deuxième longueurs d'onde $\lambda_E$ et $\lambda_{F1}$. Le réflecteur sélectif 40, par exemple un filtre spectral passe haut, est adapté pour permettre la réflexion d'ondes se propageant dans la fibre optique aux deuxième et troisième longueurs d'onde $\lambda_{F1}$, $\lambda_{F2}$ et laisse passer les ondes se propageant à la première longueur d'onde $\lambda_E$ si

l'on choisit $\lambda_{F1} < \lambda_E < \lambda_{F2}$.

Selon cette variante, une caractérisation polarimétrique de la fibre optique aux deuxième et troisièmes longueurs d'onde $\lambda_{F1}$ et $\lambda_{F2}$ est effectuée, par exemple selon les moyens décrits précédemment, afin de déterminer une matrice de Mueller associée à la fibre optique à la deuxième longueur d'onde ($\lambda_{F1}$) et une matrice de Mueller associée à la fibre optique à la troisième longueur d'onde ($\lambda_{F2}$). La mesure de la matrice de Mueller à la longueur d'onde $\lambda_{F1}$ permet de déterminer un retard de phase $\delta\varphi_{F1\_mes}$ égal au retard de phase réel $\delta\varphi_{F1}$, modulo $2\pi$. Autrement dit, le retard de phase recherché à $\lambda_{F1}$ est $\delta\varphi_{F1} = \delta\varphi_{F1\_mes} + 2m\pi$, avec $m$ entier. De même, la mesure de la matrice de Mueller à la longueur d'onde $\lambda_{F2}$ permet de déterminer un retard de phase $\delta\varphi_{F2\_mes}$ égal au retard de phase réel $\delta\varphi_{F2}$, modulo $2\pi$. Le retard de phase recherché à $\lambda_{F2}$ est $\delta\varphi_{F2} = \delta\varphi_{F2\_mes} + 2m'\pi$, avec $m'$ entier. Les deux longueurs d'onde $\lambda_{F1}$ et $\lambda_{F2}$ étant proches, le rapport $\delta\varphi_{F1}/\delta\varphi_{F2}$ est, au premier ordre, égal au rapport inverse des longueurs d'onde $\lambda_{F2}/\lambda_{F1}$. En s'assurant que les déphasages résiduels $\delta\varphi_{F1}$ et $\delta\varphi_{F2}$ restent petits, ce qui signifie que les entiers $m$ et $m'$ restent petits, typiquement inférieurs à 5, le couple ($m, m'$) permettant le respect de la condition $\delta\varphi_{F1}/\delta\varphi_{F2} = \lambda_{F2}/\lambda_{F1}$ peut être facilement identifié, ce qui permet de déduire les valeurs de $\delta\varphi_{F1}$ et $\delta\varphi_{F2}$. Dans un deuxième temps, le retard de phase $\delta\varphi_E$ à $\lambda_E$ est calculé par la règle de trois : $\delta\varphi_E = \delta\varphi_{Fi} * \lambda_{Fi}/\lambda_E$ ($i$= 1 ou 2). Il est ainsi possible de déduire les matrices de la fibre optique aller et retour à la longueur d'onde $\lambda_E$ et de déterminer la matrice recherchée de l'échantillon, comme expliqué précédemment (équation 5).

En pratique, la technologie actuelle des fibres monomodes standard présente l'inconvénient de présenter une orientation des axes neutres pas suffisamment bien définie et pouvant varier en fonction des contraintes, de la température, etc. On pourra préférer selon une variante l'utilisation de fibres à maintien de polarisation dont l'orientation des axes neutres en entrée et en sortie est connue et fixée. Cependant, il s'avère que le retard de phase introduit par une fibre optique à maintien de polarisation standard est important (typiquement $\pi$ par mm), ce qui peut s'avérer gênant pour la mise en oeuvre de la méthode selon les variantes décrites au moyen de la figure 3.

Les déposants ont développé une fibre optique monomode particulièrement avantageuse pour la mise en oeuvre de la méthode de caractérisation polarimétrique selon la présente description.

Cette fibre optique, appelée dans la suite de la description « fibre monomode à maintien de polarisation et à retard compensé », comprend deux tronçons d'une même fibre monomode à maintien de polarisation, d'égales longueurs, raccordés entre eux (par une soudure, par exemple), l'axe rapide du premier tronçon étant aligné avec l'axe lent du second tronçon. Avec cet arrangement, la direction des axes neutres de cette fibre, à l'entrée comme à la sortie, peut être facilement déterminée en la plaçant entre polariseurs croisés et en cherchant l'extinction du champ transmis. Dans cette situation en effet, la direction du polariseur d'entrée (respectivement de sortie) est celle de l'un des axes neutres de la fibre à l'entrée (respectivement à la sortie). Ainsi, si les longueurs des tronçons sont rigoureusement égales et si les deux tronçons sont conditionnés de la même manière (mêmes courbures, même température...), il est attendu que le retard de phase apporté par la fibre monomode à maintien de polarisation et à retard compensé entre les deux composantes du champ injecté soit nul ou négligeable, le deuxième tronçon compensant exactement le premier. Dans la réalité, une légère différence de longueur entre les deux fibres et/ou le conditionnement et/ou un environnement différents pour les deux tronçons peuvent induire l'existence d'un déphasage résiduel, apporté par la fibre monomode à maintien de polarisation et à retard compensé entre les deux composantes du champ à la sortie de la fibre. Les déposants ont montré que ce déphasage résiduel pouvait être inférieur à $8\pi$ quelle que soit la longueur de la fibre, voire en dessous de $4\pi$. La méthode telle que décrite précédemment permettra alors par mesure de la matrice de Mueller de la fibre optique à une deuxième longueur d'onde $\lambda_{F1}$, voire à deux longueurs d'onde $\lambda_{F1}$, $\lambda_{F2}$ comme cela a été décrit, de déterminer parfaitement la matrice de la fibre optique à la première longueur d'onde $\lambda_E$ et d'en déduire la matrice de Mueller de l'échantillon. Par ailleurs, une telle fibre présente peu d'effets chiraux.

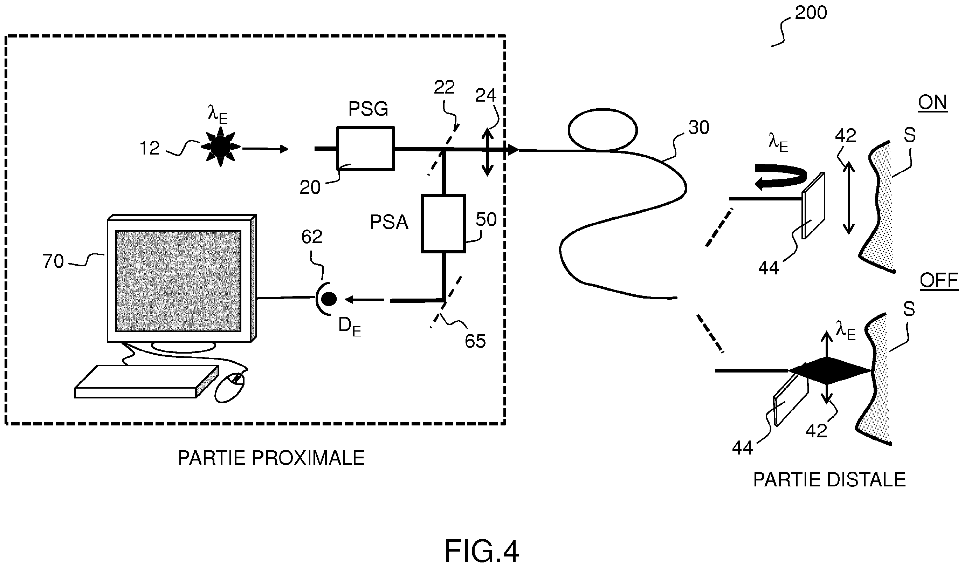

La figure 4 représente un dispositif de caractérisation polarimétrique 200 selon un deuxième exemple, pour la mise en oeuvre d'une méthode de caractérisation polarimétrique selon la présente description.

Le dispositif 200 comprend un certain nombre d'éléments identiques aux éléments du dispositif 100 et qui ne sont pas décrits à nouveau, dont notamment les PSG 20 et PSA 50, la fibre optique monomode 30, les moyens de traitement 70.

Selon cette variante, la source d'émission ne comprend plus qu'une source d'émission 12 émettant à la première longueur d'onde $\lambda_E$, par exemple une diode laser, et un détecteur en sortie du PSA sensible à cette même longueur d'onde, par exemple une photodiode.

Le réflecteur sélectif décrit sur la figure 3 est remplacé par un réflecteur amovible, par exemple un réflecteur commutable, par exemple de type MEMS, pouvant fonctionner dans un mode ON lorsqu'il est positionné en sortie de fibre et OFF lorsqu'il est écarté de l'extrémité de la fibre, comme cela est illustré sur la figure 4.

Selon cette variante, il est possible d'effectuer alternativement une caractérisation de la fibre optique directement à la longueur d'onde d'intérêt $\lambda_E$, puis une caractérisation de l'ensemble comprenant la fibre optique et l'échantillon également à cette même longueur d'onde. Si les caractérisations de la fibre et de l'ensemble fibre/échantillon sont faites dans un temps suffisamment court (typiquement inférieur à 10 ms), il est possible de mettre en oeuvre la méthode avec une simple fibre optique monomode ou une fibre optique monomode à maintien de polarisation standard. Bien entendu, cette méthode peut également être mise en oeuvre avec la fibre optique monomode à maintien de polarisation et retard compensé telle qu'elle a été décrite précédemment.

La matrice de Mueller de l'échantillon peut alors être déterminée à partir des matrices de Mueller de la fibre et de l'ensemble comprenant la fibre et l'échantillon, comme cela a été expliqué précédemment (équation 5).

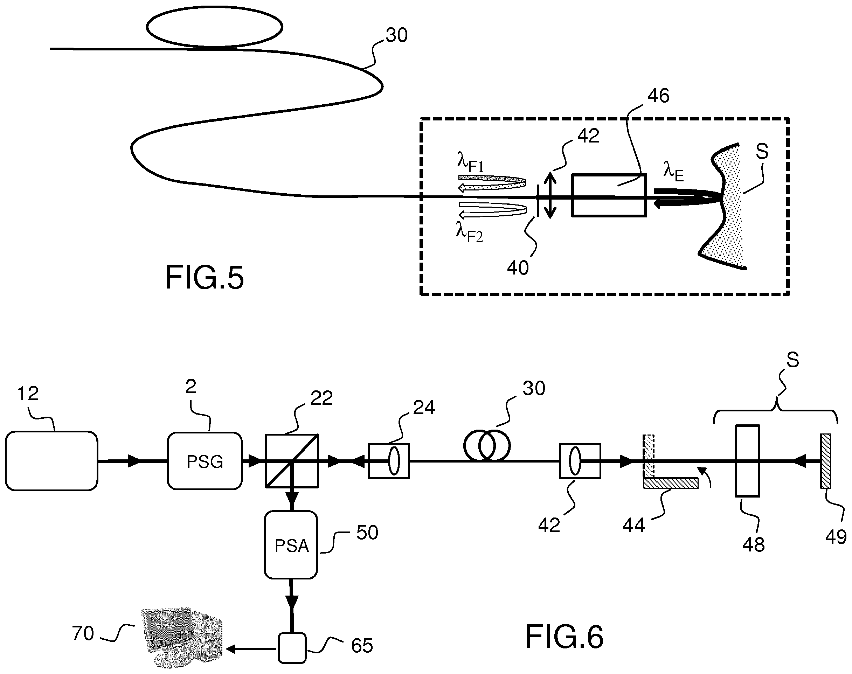

La figure 5 montre une variante applicable à l'un ou l'autre des exemples de dispositifs décrits au moyen des figures 3 et 4.

Selon cette variante, un système de balayage 46 est agencé du côté proximal, à la suite des moyens de focalisation 42. Dans autre arrangement, le système de balayage 46 peut être placé entre la fibre 30 et les moyens de focalisation 42. Dans ce cas, il peut avantageusement être précédé de moyens de collimation du faisceau sortant de la fibre 30. Le système de balayage permet de scanner l'échantillon afin de reconstruire une image d'une région d'intérêt.

Les déposants ont également montré qu'il était possible grâce à un microbalayage sur un ensemble de points voisins de l'échantillon de s'affranchir des artefacts qui pourraient résulter d'une mesure ponctuelle. En particulier, la matrice de Mueller mesurée au point de focalisation du faisceau sur un objet peut faire apparaitre un taux de dépolarisation inférieur au taux de dépolarisation qui serait obtenu en analysant une région plus large. Le taux de dépolarisation produit par une telle région plus large peut être obtenu à partir de la moyenne d'une série de matrices de Mueller mesurées ponctuellement en divers endroits de cette région.

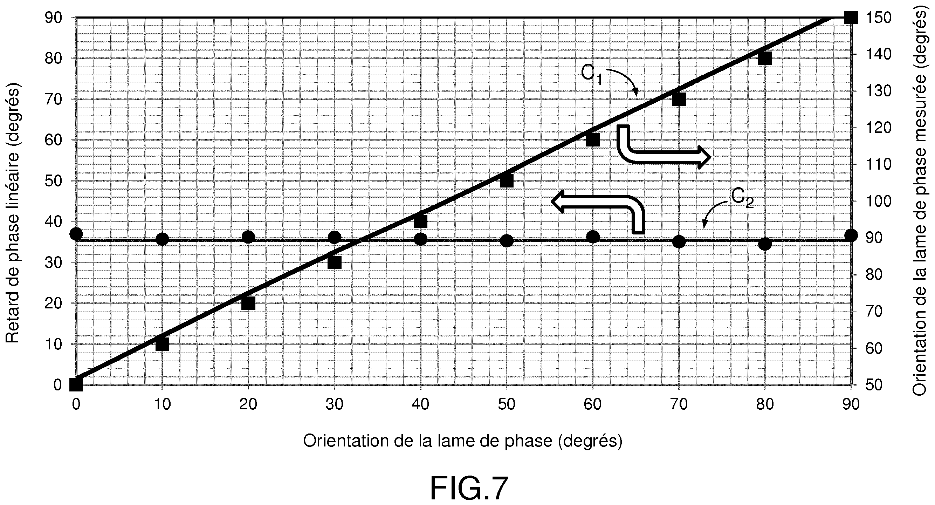

Les figures 7 à 11 montrent des premiers résultats expérimentaux obtenus avec la méthode selon la présente description.

Ces premiers résultats sont obtenus avec un dispositif du type de celui de la figure 4 et sur des échantillons « tests » bien calibrés. La figure 6 montre le montage expérimental mis en oeuvre pour ces validations. L'ensemble des éléments utilisés sont identiques à ceux représentés sur la figure 4 mais l'échantillon S est formé ici d'un objet d'analyse 48 calibré et d'un miroir plan 49.

Dans le cas des résultats montrés sur la figure 7, l'échantillon est formé d'une lame d'onde de type lame $\lambda/8$ suivie d'un miroir. La lame $\lambda/8$ introduit un retard de phase de 45° entre ses axes neutres lorsqu'elle est traversée en simple passage par la lumière. Cette lame est tournée dans le plan perpendiculaire à la direction de propagation de la lumière de manière à faire varier l'orientation de ses axes neutres par rapport au repère du laboratoire. La courbe C1 montre l'orientation donnée à la lame par rapport à une orientation de référence arbitraire, comprise entre 0° et 90° (traits pleins), et l'orientation mesurée par le dispositif (points). La courbe C2 montre, pour chacune de ces orientations, le retard de phase mesuré pour cette lame. Ce retard, égal à 90°, correspond au retard de phase accumulé lors de la double traversée de la lame $\lambda/8$ (aller et retour) analysée.

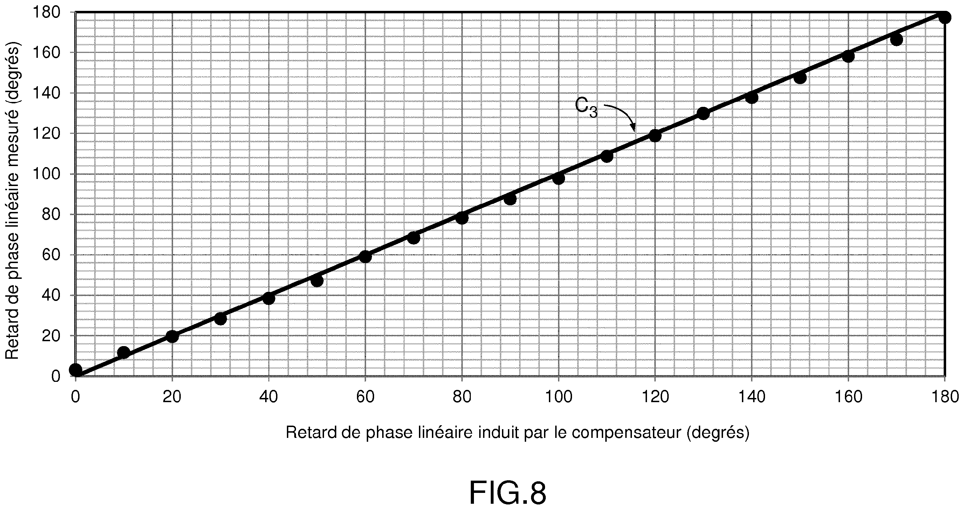

Dans le cas des résultats montrés sur la figure 8, l'échantillon est constitué d'un compensateur de Babinet-Soleil suivi d'un miroir. Le compensateur de Babinet-Soleil introduit un retard de phase connu et ajustable entre ses axes neutres lorsqu'il est traversé par la lumière. La courbe C3 montre le retard de phase linéaire introduit sur un aller-retour de la lumière ajusté par réglage du compensateur de Babinet Soleil (traits pleins) et le retard de phase mesuré correspondant (points).

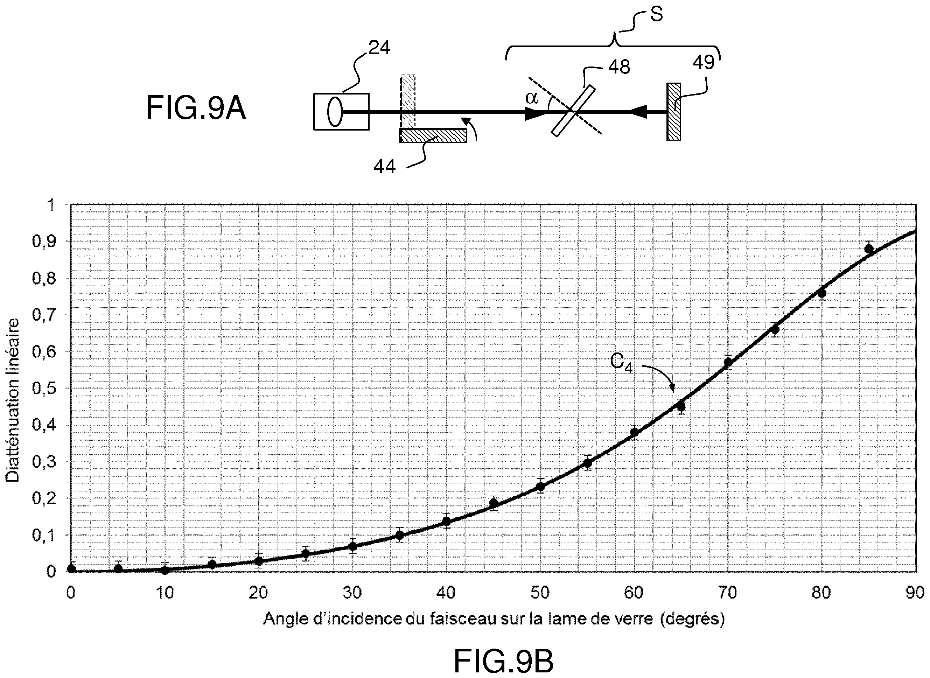

La figure 9A illustre un échantillon constitué d'une lame à faces parallèles 48 suivie d'un miroir 49. Les faces de la lame sont perpendiculaires au plan d'incidence du faisceau lumineux issu du système de collimation 24 et leur normale est inclinée d'un angle $\alpha$ ajustable par rapport à la direction du faisceau. Cette lame se comporte donc comme un composant diatténuateur linéaire pur dont la diatténuation est réglable par l'intermédiaire de l'angle $\alpha$. La figure 9B montre la diatténuation linéaire de la lame à faces parallèles sur un aller-retour de la lumière (traits pleins) calculée en fonction de l'angle $\alpha$ et la diatténuation linéaire mesurée correspondante (points). On montre par ailleurs que pour chacun de ces points, la diatténuation circulaire et le retard de phase linéaire mesurés sont nuls.

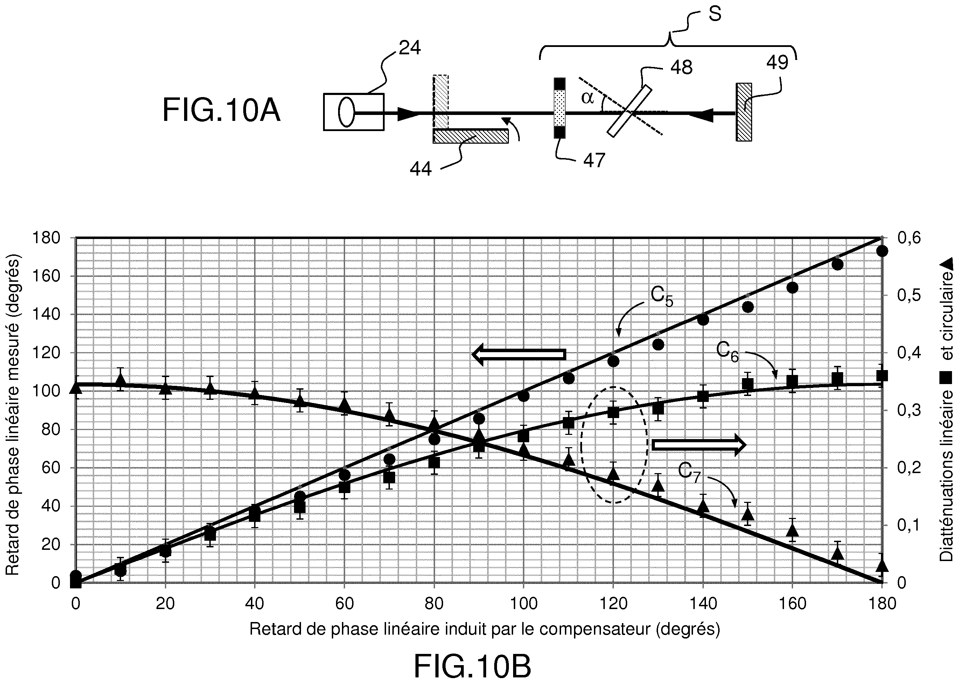

La figure 10A montre un échantillon constitué d'un compensateur de Babinet-Soleil 47, suivi d'une lame à faces parallèles 48 et d'un miroir 49. La lame à faces parallèles est positionnée par rapport au faisceau incident comme dans le cas de la figure 9A. Dans cet arrangement, la normale à la lame est inclinée par rapport au faisceau incident d'un angle $\alpha$ tel qu'elle introduit une diatténuation linéaire de 35% sur un aller-retour de la lumière. Concernant le compensateur de Babinet-Soleil, son axe rapide est orienté, dans le plan perpendiculaire au faisceau, de sorte qu'il fait un angle de 45° par rapport à l'axe de rotation de la lame. Avec cet arrangement, la lame restant fixe, le retard de phase introduit par le compensateur de Babinet est ajusté entre 0° et 90° pour une traversée simple, c'est-à-dire entre 0° et 180° sur un aller-retour de la lumière.

Les simulations numériques montrent que, lorsque le compensateur de Babinet-Soleil est réglé pour introduire un retard de phase $\gamma$ compris entre 0° et 90° lors d'une traversée simple, l'ensemble compensateur + lame à faces parallèles se comporte comme un composant introduisant un retard de phase $2\gamma$ sur un aller-retour et une combinaison de diatténuations linéaire $D_L$ et circulaire $D_C$ telles que la diatténuation résultante $D = \sqrt{D_L^2 + D_c^2} = 35\%$. Lorsque $\gamma = 0°$, la diatténuation est une diatténuation linéaire pure $D = D_L = 35\%$ et lorsque $\gamma = 90°$, la diatténuation est une diatténuation circulaire pure $D = D_C = 35\%$. Les courbes C5, C6 et C7 de la figure 10B montrent en traits pleins les résultats des simulations calculant respectivement le retard de phase, la diatténuation circulaire et la diatténuation linéaire dans l'arrangement de la figure 10A. Les points associés au courbes C5, C6 et C7 montrent les résultats des mesures expérimentales correspondantes.

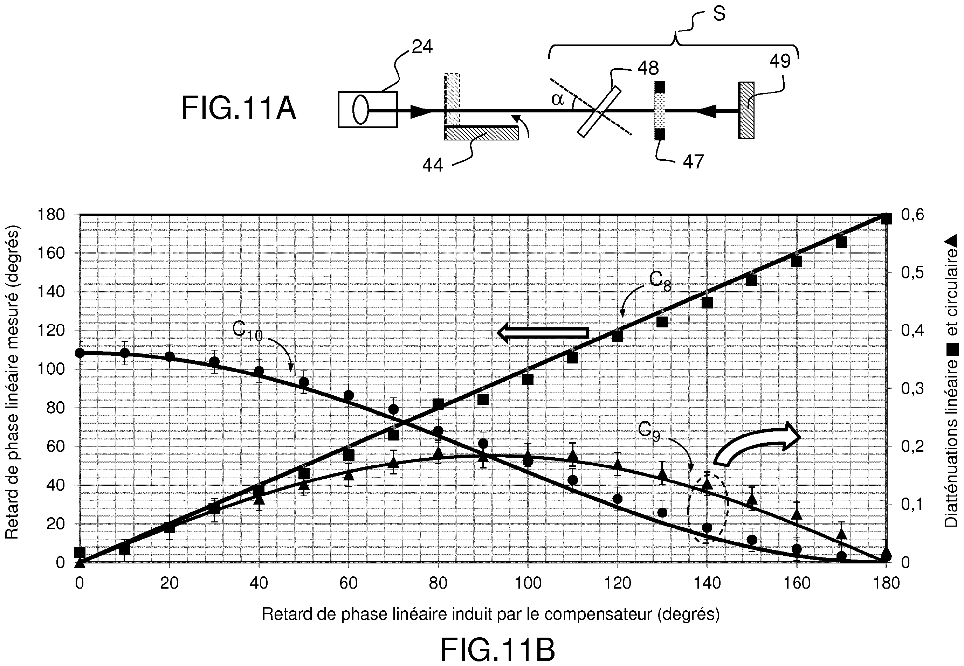

La figure 11A montre un échantillon identique à celui de la figure 10A mais dans lequel l'ordre des composants est inversé. Autrement dit, l'échantillon est constitué d'une lame à faces parallèles 48 suivie d'un compensateur de Babinet-Soleil 47 et d'un miroir 49, tous les autres réglages étant conservés identiques à ceux de la figure 10A.

Les simulations numériques montrent que, lorsque le compensateur de Babinet-Soleil est réglé pour introduire un retard de phase $\gamma$ compris entre 0° et 90° lors d'une traversée simple, l'ensemble compensateur + lame à faces parallèles se comporte comme un composant introduisant un retard de phase $2\gamma$ sur un aller-retour et une combinaison de diatténuations linéaire $D_L$ et circulaire $D_C$ telles que la diatténuation linéaire décroit de 35% à 0% lorsque le retard de phase $\gamma$ évolue de 0° à 90°, et la diatténuation circulaire croit de 0% jusqu'à 17,5% puis décroit jusqu'à 0% lorsque le retard de phase $\gamma$ évolue de 0° à 90°. Les courbes C8, C9 et C10 de la figure 11B montrent en traits pleins les résultats des simulations calculant respectivement le retard de phase, la diatténuation circulaire et la diatténuation linéaire dans l'arrangement de la figure 11A. Les points associés au courbes C8, C9 et C10 montrent les résultats des mesures expérimentales correspondantes.

Les résultats expérimentaux ainsi présentés montrent la faisabilité de la méthode de caractérisation polarimétrique selon la présente description et les possibilités d'accéder à des informations polarimétriques concernant l'échantillon complètes et de façon précise, notamment les retards de phase linéaire et les diatténuations linéaire et circulaire.

La méthode ainsi décrite pourra être mise en oeuvre non seulement pour la caractérisation polarimétrique d'échantillons biologiques en endoscopie mais aussi pour la caractérisation d'échantillons difficiles d'accès, comme par exemple la caractérisation de matériaux isolants ou conducteurs en environnement hostile (présence de radiations nucléaires, champs électromagnétiques forts, très hautes ou très basses températures, etc.).

Bien que décrits à travers un certain nombre d'exemples de réalisation détaillés, le procédé et le dispositif de caractérisation polarimétrique selon l'invention comprennent différentes variantes, modifications et perfectionnements qui apparaîtront de façon évidente à l'homme de l'art, étant entendu que ces différentes variantes, modifications et perfectionnements font partie de la portée de l'invention, telle que définie par les revendications qui suivent.

**Revendications**

1. Dispositif de caractérisation polarimétrique déportée (100, 200) d'un échantillon (S) comprenant :

   - une source d'émission (10) d'au moins une onde lumineuse incidente à au moins une première longueur d'onde ($\lambda_E$) ;
   - une fibre optique monomode (30) dans laquelle l'onde lumineuse incidente est destinée à se propager;
   - un générateur d'états de polarisation (20) agencé du côté proximal de la fibre optique et permettant la génération d'un nombre donné d'états de polarisation de l'onde lumineuse incidente, dits états sonde;
   - un réflecteur (40, 44) destiné à être agencé du côté distal de la fibre optique ;
   - un analyseur d'états de polarisation (50) agencé du côté proximal de la fibre optique et permettant, pour chaque état sonde de l'onde incidente, l'analyse de la polarisation de l'onde lumineuse obtenue après propagation de l'onde incidente dans la fibre optique (30), réflexion du côté distal de la fibre optique et propagation inverse dans la fibre optique (30);
   - des moyens de traitement (70) permettant de déterminer :

     ◦ à partir d'une première caractérisation polarimétrique de la fibre optique, obtenue par l'analyse pour chaque état sonde, de la polarisation d'au moins une onde réfléchie du côté distale de la fibre optique au moyen du réflecteur et propagée en sens inverse dans la fibre optique (30), une matrice de Mueller ($\mathbf{M_F}(\lambda_E)$) associée à la fibre optique à la première longueur d'onde ;
     ◦ à partir d'une deuxième caractérisation polarimétrique de l'ensemble comprenant la fibre optique et l'échantillon, obtenue par l'analyse pour chaque état sonde de la polarisation, d'une onde renvoyée du côté distal de la fibre par l'échantillon et propagée en sens inverse dans la fibre optique (30), une matrice de Mueller ($\mathbf{M_T}$) associée audit ensemble à la première longueur d'onde ;
     ◦ à partir des matrices de Mueller associées respectivement à la fibre optique et à l'ensemble comprenant la fibre optique et l'échantillon, la matrice de Mueller ($\mathbf{M_O}$) associée à l'échantillon à la première longueur d'onde.

2. Dispositif de caractérisation polarimétrique selon la revendication 1, dans lequel :

- La source d'émission permet l'émission d'une onde à la première longueur d'onde ($\lambda_E$) et l'émission d'une onde à une deuxième longueur d'onde ($\lambda_{F1}$) distincte de la première longueur d'onde,
- le réflecteur est un réflecteur spectral permettant la réflexion d'une onde se propageant dans la fibre optique à la deuxième longueur d'onde ($\lambda_{F1}$) pour la caractérisation polarimétrique de la fibre optique à la deuxième longueur d'onde ($\lambda_{F1}$) et le passage de l'onde à la première longueur d'onde ($\lambda_E$) pour la caractérisation polarimétrique de l'ensemble comprenant la fibre optique et l'échantillon à la première longueur d'onde ($\lambda_E$) ;
- les moyens de traitement permettent de déterminer :

  ◦ à partir de la caractérisation polarimétrique de la fibre optique à la deuxième longueur d'onde ($\lambda_{F1}$), une matrice de Mueller ($\mathbf{M_F}(\lambda_{F1})$) associée à la fibre optique à la deuxième longueur d'onde ($\lambda_{F1}$);
  ◦ à partir de la matrice de Mueller associée à la fibre optique à la deuxième longueur d'onde ($\lambda_{F1}$), une matrice de Mueller ($\mathbf{M_F}(\lambda_E)$) associée à la fibre optique à la première longueur d'onde ($\lambda_E$).

3. Dispositif de caractérisation polarimétrique selon la revendication 2, dans lequel :

- La source d'émission permet en outre l'émission d'une onde à une troisième longueur d'onde ($\lambda_{F2}$) distincte des première et deuxième longueurs d'onde,
- le réflecteur spectral permet la réflexion d'ondes se propageant dans la fibre optique aux deuxième et troisième longueurs d'onde ($\lambda_{F1}$, $\lambda_{F2}$) pour la caractérisation polarimétrique de la fibre optique aux deuxième et troisième longueurs d'onde ($\lambda_{F1}$, $\lambda_{F2}$);
- les moyens de traitement permettent de déterminer :

  ◦ à partir d'une caractérisation polarimétrique de la fibre optique à la deuxième longueur d'onde ($\lambda_{F1}$) et à partir d'une caractérisation polarimétrique de la fibre optique à la troisième longueur d'onde ($\lambda_{F2}$), respectivement une matrice de Mueller ($\mathbf{M_F}(\lambda_{F1})$) associée à la fibre optique à la deuxième longueur d'onde ($\lambda_{F1}$) et une matrice de Mueller ($\mathbf{M_F}(\lambda_{F2})$) associée à la fibre optique à la troisième longueur d'onde ($\lambda_{F2}$) ;
  ◦ à partir des matrices de Mueller associées à la fibre optique aux deuxième longueur d'onde ($\lambda_{F1}$) et troisième longueur d'onde ($\lambda_{F2}$), la matrice de Mueller ($\mathbf{M_F}(\lambda_E)$) associée à la fibre optique à la première longueur d'onde ($\lambda_E$).

4. Dispositif de caractérisation polarimétrique selon la revendication 1, dans lequel le réflecteur est un réflecteur commutable entre une position réfléchissante et une position passante, permettant, dans la position réfléchissante, la réflexion d'une onde se propageant dans la fibre optique à la première longueur d'onde pour la caractérisation polarimétrique de la fibre optique et, dans la position passante, la réflexion de l'onde par l'échantillon pour la caractérisation polarimétrique de l'ensemble comprenant la fibre optique et l'échantillon.

5. Dispositif selon l'une quelconque des revendications précédentes, dans lequel la fibre optique monomode (30) est une fibre optique à maintien de polarisation.

6. Dispositif selon la revendication 5, dans lequel la fibre optique monomode comprend un premier tronçon d'une fibre optique monomode à maintien de polarisation et un deuxième tronçon de la même fibre optique monomode à maintien de polarisation, les tronçons étant de même longueur et raccordés entre eux de telle sorte que l'axe rapide du premier tronçon soit aligné avec l'axe lent du deuxième tronçon et inversement.

7. Dispositif selon l'une quelconque des revendications précédentes, comprenant en outre du côté distal de la fibre optique, des moyens de focalisation (42) d'une onde à la première longueur d'onde pour la caractérisation d'une zone ponctuelle de l'échantillon.

8. Dispositif selon la revendication 7, comprenant en outre du côté distal de la fibre optique, des moyens de balayage (46) pour la caractérisation polarimétrique d'un ensemble de zones ponctuelles de l'échantillon.

9. Méthode de caractérisation polarimétrique déportée d'un échantillon (S) comprenant :

- l'émission d'une onde lumineuse incidente à au moins une première longueur d'onde ($\lambda_E$) destinée à se propager dans une fibre optique monomode (30);
- la caractérisation polarimétrique de la fibre optique à la première longueur d'onde, comprenant :

  ◦ la génération d'un nombre donné d'états de polarisation de l'onde lumineuse incidente, appelés états sonde, au moyen d'un générateur d'états de polarisation (20) agencé du côté proximal de la fibre optique ;
○ l'analyse, au moyen d'un analyseur d'états de polarisation (50) agencé du côté proximal de la fibre optique, pour chaque état sonde de l'onde incidente, de la polarisation de l'onde lumineuse obtenue après propagation de l'onde incidente dans la fibre optique (30), réflexion au moyen d'un réflecteur (40, 44) agencé du côté distal de la fibre optique et propagation inverse dans la fibre optique (30) ;
○ la détermination d'une matrice de Mueller ($\mathbf{M_F}(\lambda_E)$) associée à la fibre optique à la première longueur d'onde ;

- la caractérisation polarimétrique de l'ensemble comprenant la fibre optique et l'échantillon à la première longueur d'onde, comprenant :

○ au moyen desdits générateur d'états de polarisation (20) et analyseur d'états de polarisation (50), l'analyse pour chaque état sonde de la polarisation, d'une onde renvoyée du côté distal de la fibre par l'échantillon et propagée en sens inverse dans la fibre optique (30) ;
○ la détermination d'une matrice de Mueller ($\mathbf{M_T}$) associée audit ensemble à la première longueur d'onde ;

- la détermination de la matrice de Mueller ($\mathbf{M_O}$) associée à l'échantillon à la première longueur d'onde à partir des matrices de Mueller associées respectivement à la fibre optique et à l'ensemble comprenant la fibre optique et l'échantillon.

**10.** Méthode de caractérisation polarimétrique selon la revendication 9, comprenant :

- l'émission d'une onde lumineuse à une deuxième longueur d'onde ($\lambda_{F1}$) distincte de la première longueur d'onde ($\lambda_E$),

et dans laquelle :

- le réflecteur est un réflecteur spectral permettant la réflexion d'une onde se propageant dans la fibre optique à la deuxième longueur d'onde ($\lambda_{F1}$) pour la caractérisation polarimétrique de la fibre optique à la deuxième longueur d'onde ($\lambda_{F1}$) et le passage de l'onde à la première longueur d'onde ($\lambda_E$) pour la caractérisation polarimétrique de l'ensemble comprenant la fibre optique et l'échantillon à la première longueur d'onde ($\lambda_E$) ;
- la détermination de la matrice de Mueller ($\mathbf{M_F}(\lambda_E)$) associée à la fibre optique à la première longueur d'onde comprend :

○ à partir de la caractérisation polarimétrique de la fibre optique à la deuxième longueur d'onde ($\lambda_{F1}$), une matrice de Mueller ($\mathbf{M_F}(\lambda_{F1})$) associée à la fibre optique à la deuxième longueur d'onde ($\lambda_{F1}$);
○ à partir de la matrice de Mueller associée à la fibre optique à la deuxième longueur d'onde ($\lambda_{F1}$), une matrice de Mueller ($\mathbf{M_F}(\lambda_E)$) associée à la fibre optique à la première longueur d'onde ($\lambda_E$).

**11.** Méthode de caractérisation polarimétrique selon la revendication 10, comprenant :

- l'émission d'une onde à une troisième longueur d'onde ($\lambda_{F2}$) distincte des première et deuxième longueurs d'onde,

et dans laquelle :

- le réflecteur spectral permet la réflexion d'ondes se propageant dans la fibre optique aux deuxième et troisième longueurs d'onde ($\lambda_{F1}$, $\lambda_{F2}$) pour la caractérisation polarimétrique de la fibre optique aux deuxième et troisième longueurs d'onde ($\lambda_{F1}$, $\lambda_{F2}$);
- la détermination de la matrice de Mueller ($\mathbf{M_F}(\lambda_E)$) associée à la fibre optique à la première longueur d'onde comprend :

○ à partir d'une caractérisation polarimétrique de la fibre optique à la deuxième longueur d'onde ($\lambda_{F1}$) et à partir d'une caractérisation polarimétrique de la fibre optique à la troisième longueur d'onde ($\lambda_{F2}$), respectivement une matrice de Mueller ($\mathbf{M_F}(\lambda_{F1})$) associée à la fibre optique à la deuxième longueur d'onde ($\lambda_{F1}$) et une matrice de Mueller ($\mathbf{M_F}(\lambda_{F2})$) associée à la fibre optique à la troisième longueur d'onde ($\lambda_{F2}$) ;
○ à partir des matrices de Mueller associées à la fibre optique aux deuxième longueur d'onde ($\lambda_{F1}$) et troisième longueur d'onde ($\lambda_{F2}$), la matrice de Mueller ($\mathbf{M_F}(\lambda_E)$) associée à la fibre optique à la première

longueur d'onde ($\lambda_E$).

**12.** Méthode de caractérisation polarimétrique selon la revendication 9, dans lequel le réflecteur est un réflecteur commutable entre une position réfléchissante et une position passante, permettant, dans la position réfléchissante, la réflexion d'une onde se propageant dans la fibre optique à la première longueur d'onde pour la caractérisation polarimétrique de la fibre optique et, dans la position passante, la réflexion de l'onde par l'échantillon pour la caractérisation polarimétrique de l'ensemble comprenant la fibre optique et l'échantillon.

**13.** Méthode selon l'une quelconque des revendications 9 à 12, comprenant en outre du côté distal de la fibre optique, la focalisation d'une onde lumineuse à la première longueur d'onde aux moyens de focalisation (42) pour la caractérisation d'une zone ponctuelle de l'échantillon.

**14.** Méthode selon la revendication 13, comprenant en outre du côté distal de la fibre optique, le balayage par des moyens de balayage (46) de l'onde lumineuse focalisée pour la caractérisation polarimétrique d'un ensemble de zones ponctuelles de l'échantillon.

**Patentansprüche**

**1.** Vorrichtung zur polarimetrischen Ferncharakterisierung (100, 200) einer Probe (S), umfassend:

- eine Emissionsquelle (10) von mindestens einer einfallenden Lichtwelle bei mindestens einer ersten Wellenlänge ($\lambda_E$);
- eine monomodische optische Faser (30), in welcher die einfallende Lichtwelle dazu bestimmt ist, sich auszubreiten;
- ein Generator von Polarisationszuständen (20), der an der proximalen Seite der optischen Faser angeordnet ist und der die Erzeugung einer gegebenen Anzahl von Polarisationszuständen der einfallenden Lichtwelle, so genannte Sondenzustände, ermöglicht;
- ein Reflektor (40, 44) der dazu bestimmt ist, an der distalen Seite der optischen Faser angeordnet zu sein;
- ein Analysator von Polarisationszuständen (50), der an der proximalen Seite der optischen Faser angeordnet ist und der, für jeden Sondenzustand der einfallenden Welle, die Analyse der Polarisation der Lichtwelle ermöglicht, die nach Ausbreitung der einfallenden Welle in der optischen Faser (30), Reflexion an der distalen Seite der optischen Faser und entgegengesetzter Ausbreitung in der optischen Faser (30) erhalten wurde;
- Mittel zur Behandlung (70), die es ermöglichen zu bestimmen:

  ◦ ausgehend von einer ersten polarimetrischen Charakterisierung der optischen Faser, erhalten durch die Analyse für jeden Sondenzustand, der Polarisation von mindestens einer an der distalen Seite der optischen Faser mittels des Reflektors reflektierten und in entgegengesetzter Richtung in der optischen Faser (30) ausgebreiteten Welle, eine Müller-Matrix ($\mathbf{M_F}(\lambda_E)$), die mit der optischen Faser bei der ersten Wellenlänge assoziiert ist;
  ◦ ausgehend von einer zweiten polarimetrischen Charakterisierung der die optische Faser und die Probe umfassenden Einheit, erhalten durch die Analyse für jeden Sondenzustand der Polarisation, von einer an der distalen Seite der Faser durch die Probe zurückgeworfenen und in entgegengesetzter Richtung in der optischen Faser (30) ausgebreiteten Welle, eine Müller-Matrix ($\mathbf{M_T}$), die mit der besagten Einheit bei der ersten Wellenlänge assoziiert ist;
  ◦ ausgehend von den Müller-Matrizen, die jeweils mit der optischen Faser und mit der die optische Faser und die Probe umfassenden Einheit assoziiert sind, die Müller-Matrix ($\mathbf{M_O}$), die mit der Probe bei der ersten Wellenlänge assoziiert ist.

**2.** Vorrichtung zur polarimetrischen Charakterisierung nach Anspruch 1, in welcher:

- Die Emissionsquelle die Emission einer Welle bei der ersten Wellenlänge ($\lambda_E$) und die Emission einer Welle bei einer zweiten Wellenlänge ($\lambda_{F1}$), die von der ersten Wellenlänge verschieden ist, ermöglicht,
- der Reflektor ein Spektralreflektor ist, der die Reflexion einer Welle, die sich in der optischen Faser bei der zweiten Wellenlänge ($\lambda_{F1}$) für die polarimetrische Charakterisierung der optischen Faser bei der zweiten Wellenlänge ($\lambda_{F1}$) ausbreitet, und den Durchgang der Welle bei der ersten Wellenlänge ($\lambda_E$) für die polarimetrische Charakterisierung der die optische Faser und die Probe umfassenden Einheit bei der ersten Wellenlänge ($\lambda_E$) ermöglicht;

- die Mittel zur Behandlung es ermöglichen zu bestimmen:

◦ ausgehend von der polarimetrischen Charakterisierung der optischen Faser bei der zweiten Wellenlänge ($\lambda_{F1}$), eine Müller-Matrix ($\mathbf{M_F}(\lambda_{F1})$), die mit der optischen Faser bei der zweiten Wellenlänge ($\lambda_{F1}$) assoziiert ist;

◦ ausgehend von der Müller-Matrix, die mit der optischen Faser bei der zweiten Wellenlänge ($\lambda_{F1}$) assoziiert ist, eine Müllermatrix ($\mathbf{M_F}(\lambda_E)$), die mit der optischen Faser bei der ersten Wellenlänge ($\lambda_E$) assoziiert ist.

3. Vorrichtung zur polarimetrischen Charakterisierung nach Anspruch 2, in welcher:

- Die Emissionsquelle des Weiteren die Emission einer Welle bei einer dritten Wellenlänge ($\lambda_{F2}$) ermöglicht, die von der ersten und der zweiten Wellenlänge verschieden ist,
- der Spektralreflektor die Reflexion von Wellen ermöglicht, die sich in der optischen Faser bei den zweiten und dritten Wellenlängen ($\lambda_{F1}$, $\lambda_{F2}$) für die polarimetrische Charakterisierung der optischen Faser bei den zweiten und dritten Wellenlängen ($\lambda_{F1}$, $\lambda_{F2}$) ausbreiten;
- die Mittel zur Behandlung es ermöglichen zu bestimmen:

◦ ausgehend von einer polarimetrischen Charakterisierung der optischen Faser bei der zweiten Wellenlänge ($\lambda_{F1}$) und ausgehend von einer polarimetrischen Charakterisierung der optischen Faser bei der dritten Wellenlänge ($\lambda_{F2}$), jeweils eine Müller-Matrix ($\mathbf{M_F}(\lambda_{F1})$), die mit der optischen Faser bei der zweiten Wellenlänge ($\lambda_{F1}$) assoziiert ist, und eine Müller-Matrix ($\mathbf{M_F}(\lambda_{F2})$), die mit der optischen Faser bei der dritten Wellenlänge ($\lambda_{F2}$) assoziiert ist;

◦ ausgehend von den Müller-Matrizen, die mit der optischen Faser bei der zweiten Wellenlänge ($\lambda_{F1}$) und bei der dritten Wellenlänge ($\lambda_{F2}$) assoziiert sind, die Müller-Matrix ($\mathbf{M_F}(\lambda_E)$), die mit der optischen Faser bei der ersten Wellenlänge ($\lambda_E$) assoziiert ist.

4. Vorrichtung zur polarimetrischen Charakterisierung nach Anspruch 1, in welcher der Reflektor ein zwischen einer reflektierenden Position und einer durchgehenden Position umschaltbarer Reflektor ist, der in der reflektierenden Position die Reflexion einer Welle, die sich in der optischen Faser bei der ersten Wellenlänge für die polarimetrische Charakterisierung der optischen Faser ausbreitet, und in der durchgehenden Position die Reflexion der Welle durch die Probe für die polarimetrische Charakterisierung der die optische Faser und die Probe umfassenden Einheit ermöglicht.

5. Vorrichtung nach einem der vorstehenden Ansprüche, in welcher die monomodische optische Faser (30) eine optische Faser zur Polarisationserhaltung ist.

6. Vorrichtung nach Anspruch 5, in welcher die monomodische optische Faser einen ersten Abschnitt einer monomodischen optischen Faser zur Polarisationserhaltung und einen zweiten Abschnitt derselben monomodischen optischen Faser zur Polarisationserhaltung umfasst, wobei die Abschnitte die gleiche Länge haben und dergestalt miteinander verbunden sind, dass die lichtstarke Achse des ersten Abschnitts auf die lichtschwache Achse des zweiten Abschnitts und umgekehrt ausgerichtet ist.

7. Vorrichtung nach einem der vorstehenden Ansprüche, die des Weiteren an der distalen Seite der optischen Faser Mittel zum Fokussieren (42) einer Welle bei einer ersten Wellenlänge für die Charakterisierung eines punktförmigen Bereichs der Probe umfasst.

8. Vorrichtung nach Anspruch 7, die des Weiteren an der distalen Seite der optischen Faser Mittel zum Abtasten (46) für die polarimetrische Charakterisierung einer Gesamtheit von punktförmigen Bereichen der Probe umfasst.

9. Verfahren zur polarimetrischen Ferncharakterisierung einer Probe (S), das umfasst:

- die Emission einer einfallenden Lichtwelle bei mindestens einer ersten Wellenlänge ($\lambda_E$), die dazu bestimmt ist, sich in einer monomodischen optischen Faser (30) auszubreiten;
- wobei die polarimetrische Charakterisierung der optischen Faser bei der ersten Wellenlänge umfasst:

◦ die Erzeugung einer gegebenen Anzahl von Polarisationszuständen der einfallenden Lichtwelle, so genannte Sondenzustände, mittels eines Generators von Polarisationszuständen (20), der an der proximalen Seite der optischen Faser angebracht ist;

∘ die Analyse, mittels eines Analysators von Polarisationszuständen (50), der an der proximalen Seite der optischen Faser angebracht ist, für jeden Sondenzustand der einfallenden Welle, der Polarisation der Lichtwelle, die erhalten wird nach Ausbreitung der einfallenden Welle in der optischen Faser (30), Reflexion mittels eines an der distalen Seite der optischen Faser angebrachten Reflektors (40, 44), und entgegengesetzter Ausbreitung in der optischen Faser (30);

∘ die Bestimmung einer Müller-Matrix ($M_F(\lambda_E)$), die mit der optischen Faser bei der ersten Wellenlänge assoziiert ist;

- die polarimetrische Charakterisierung der die optische Faser und die Probe umfassenden Einheit bei der ersten Wellenlänge, umfassend:

∘ mittels des besagten Generators von Polarisationszuständen (20) und des besagten Analysators von Polarisationszuständen (50),

die Analyse für jeden Sondenzustand der Polarisation, von einer an der distalen Seite der Faser durch die Probe zurückgeworfenen und in entgegengesetzter Richtung in der optischen Faser (30) ausgebreiteten Welle;

∘ die Bestimmung einer Müller-Matrix ($M_T$), die mit der besagten Einheit bei der ersten Wellenlänge assoziiert ist;

- die Bestimmung der Müller-Matrix ($M_O$), die mit der Probe bei der ersten Wellenlänge assoziiert ist, ausgehend von den Müller-Matrizen, die jeweils mit der optischen Faser und mit der die optische Faser und die Probe umfassenden Einheit assoziiert sind.

10. Verfahren zur polarimetrischen Charakterisierung nach Anspruch 9, umfassend:

- die Emission einer Lichtwelle bei einer zweiten Wellenlänge ($\lambda_{F1}$), die von der ersten Wellenlänge ($\lambda_E$) verschieden ist,

und in welchem:

- der Reflektor ein Spektralreflektor ist, der die Reflexion einer Welle, die sich in der optischen Faser bei der zweiten Wellenlänge ($\lambda_{F1}$) für die polarimetrische Charakterisierung der optischen Faser bei der zweiten Wellenlänge ($\lambda_{F1}$) ausbreitet, und den Durchgang der Welle bei der ersten Wellenlänge ($\lambda_E$) für die polarimetrische Charakterisierung der die optische Faser und die Probe umfassenden Einheit bei der ersten Wellenlänge ($\lambda_E$) ermöglicht;

- die Bestimmung der Müller-Matrix ($M_F(\lambda_E)$), die mit der optischen Faser bei der ersten Wellenlänge assoziiert ist, Folgendes umfasst:

∘ ausgehend von der polarimetrischen Charakterisierung der optischen Faser bei der zweiten Wellenlänge ($\lambda_{F1}$), eine Müller-Matrix ($M_F(\lambda_{F1})$), die mit der optischen Faser bei der zweiten Wellenlänge ($\lambda_{F1}$) assoziiert ist;

∘ ausgehend von der Müller-Matrix, die mit der optischen Faser bei der zweiten Wellenlänge ($\lambda_{F1}$) assoziiert ist, eine Müller-Matrix ($M_F(\lambda_E)$), die mit der optischen Faser bei der ersten Wellenlänge ($\lambda_E$) assoziiert ist.

11. Verfahren zur polarimetrischen Charakterisierung nach Anspruch 10, umfassend:

- die Emission einer Welle bei einer dritten Wellenlänge ($\lambda_{F2}$), die von der ersten und der zweiten Wellenlänge verschieden ist,

und in welchem:

- der Spektralreflektor die Reflexion von Wellen ermöglicht, die sich in der optischen Faser bei der zweiten und dritten Wellenlänge ($\lambda_{F1}$, $\lambda_{F2}$) für die polarimetrische Charakterisierung der optischen Faser bei der zweiten und dritten Wellenlänge ($\lambda_{F1}$, $\lambda_{F2}$) ausbreiten;

- die Bestimmung der Müller-Matrix ($M_F(\lambda_E)$), die mit der optischen Faser bei der ersten Wellenlänge assoziiert ist, Folgendes umfasst:

∘ ausgehend von einer polarimetrischen Charakterisierung der optischen Faser bei der zweiten Wellenlänge

($\lambda_{F1}$) und ausgehend von einer polarimetrischen Charakterisierung der optischen Faser bei der dritten Wellenlänge ($\lambda_{F2}$), jeweils eine Müller-Matrix ($\mathbf{M_F}(\lambda_{F1})$), die mit der optischen Faser bei der zweiten Wellenlänge ($\lambda_{F1}$) assoziiert ist, und eine Müller-Matrix ($\mathbf{M_F}(\lambda_{F2})$), die mit der optischen Faser bei der dritten Wellenlänge ($\lambda_{F2}$) assoziiert ist;

◦ ausgehend von den Müller-Matrizen, die mit der optischen Faser bei der zweiten Wellenlänge ($\lambda_{F1}$) und bei der dritten Wellenlänge ($\lambda_{F2}$) assoziiert sind, die Müller-Matrix ($\mathbf{M_F}(\lambda_E)$), die mit der optischen Faser bei der ersten Wellenlänge ($\lambda_E$) assoziiert ist.

12. Verfahren zur polarimetrischen Charakterisierung nach Anspruch 9, in welchem der Reflektor ein zwischen einer reflektierenden Position und einer durchgehenden Position umschaltbarer Reflektor ist, der in der reflektierenden Position die Reflexion einer sich in der optischen Faser bei der ersten Wellenlänge für die polarimetrische Charakterisierung der optischen Faser ausbreitenden Welle und, in der durchgehenden Position, die Reflexion der Welle durch die Probe für die polarimetrische Charakterisierung der die optische Faser und die Probe umfassenden Einheit ermöglicht.

13. Verfahren nach einem der Ansprüche 9 bis 12, umfassend des Weiteren an der distalen Seite der optischen Faser die Fokussierung einer Lichtwelle bei der ersten Wellenlänge mit Mitteln zum Fokussieren (42) für die Charakterisierung eines punktförmigen Bereichs der Probe.

14. Verfahren nach Anspruch 13, umfassend des Weiteren an der distalen Seite der optischen Faser die Abtastung durch Mittel zum Abtasten (46) der Lichtwelle, die für die polarimetrische Charakterisierung einer Gesamtheit von punktförmigen Bereichen der Probe fokussiert wird.

**Claims**

1. A device for remote polarimetric characterization (100, 200) of a sample (S) comprising:

   - a source of emission (10) of at least one incident light wave at at least one first wavelength ($\lambda_E$);
   - a single-mode optical fiber (30) in which the incident light wave is intended to be propagated;
   - a polarization state generator (20) arranged on the proximal side of the optical fiber and making it possible to generate a given number of polarization states of the incident light wave, called probe states;
   - a reflector (40, 44) intended to be arranged on the distal side of the optical fiber;
   - a polarization state analyzer (50) arranged on the proximal side of the optical fiber and allowing, for each probe state of the incident wave, the analysis of the polarization of the light wave obtained after propagation of the incident wave in the optical fiber (30), reflection on the distal side of the optical fiber and back propagation in the optical fiber (30);
   - processing means (70) making it possible to determine:

     ◦ a Mueller matrix ($\mathbf{M_F}(\lambda_E)$) associated with the optical fiber at the first wavelength from a first polarimetric characterization of the optical fiber, obtained by analysis for each probe state, of the polarization of at least one wave reflected on the distal side of the optical fiber by means of the reflector and propagated in the back direction in the optical fiber (30);
     ◦ a Mueller matrix ($\mathbf{M_T}$) associated with said assembly at the first wavelength from a second polarimetric characterization of the assembly comprising the optical fiber and the sample, obtained by analysis for each probe state of the polarization, of a wave returned from the distal side of the fiber by the sample and propagated in the back direction in the optical fiber (30);
     ◦ the Mueller matrix ($\mathbf{M_O}$) associated with the sample at the first wavelength from Mueller matrices associated respectively with the optical fiber and with the assembly comprising the optical fiber and the sample.

2. The polarimetric characterization device as claimed in claim 1, wherein:

   - the source of emission allows the emission of a wave at the first wavelength ($\lambda_E$) and the emission of a wave at a second wavelength ($\lambda_{F1}$) distinct from the first wavelength,
   - the reflector is a spectral reflector allowing the reflection of a wave being propagated in the optical fiber at the second wavelength ($\lambda_{F1}$) for the polarimetric characterization of the optical fiber at the second wavelength ($\lambda_{F1}$) and the passage of the wave at the first wavelength ($\lambda_E$) for the polarimetric characterization of the assembly comprising the optical fiber and the sample at the first wavelength ($\lambda_E$);

- the processing means make it possible to determine:

  ∘ from the polarimetric characterization of the optical fiber at the second wavelength ($\lambda_{F1}$), a Mueller matrix ($\mathbf{M_F}(\lambda_{F1})$) associated with the optical fiber at the second wavelength ($\lambda_{F1}$);
  ∘ from the Mueller matrix associated with the optical fiber at the second wavelength ($\lambda_{F1}$), a Mueller matrix ($\mathbf{M_F}(\lambda_E)$) associated with the optical fiber at the first wavelength ($\lambda_E$).

3. The polarimetric characterization device as claimed in claim 2, wherein:

   - the source of emission also allows the emission of a wave at a third wavelength ($\lambda_{F2}$) distinct from the first and second wavelengths,
   - the spectral reflector allows the reflection of waves being propagated in the optical fiber at the second and third wavelengths ($\lambda_{F1}$, $\lambda_{F2}$) for the polarimetric characterization of the optical fiber at the second and third wavelengths ($\lambda_{F1}$, $\lambda_{F2}$);
   - the processing means make it possible to determine:

     ∘ from a polarimetric characterization of the optical fiber at the second wavelength ($\lambda_{F1}$) and from a polarimetric characterization of the optical fiber at the third wavelength ($\lambda_{F2}$), respectively a Mueller matrix ($\mathbf{M_F}(\lambda_{F1})$) associated with the optical fiber at the second wavelength ($\lambda_{F1}$) and a Mueller matrix ($\mathbf{M_F}(\lambda_{F2})$) associated with the optical fiber at the third wavelength ($\lambda_{F2}$);
     ∘ from the Mueller matrices associated with the optical fiber at the second wavelength ($\lambda_{F1}$) and third wavelength ($\lambda_{F2}$), the Mueller matrix ($\mathbf{M_F}(\lambda_E)$) associated with the optical fiber at the first wavelength ($\lambda_E$).

4. The polarimetric characterization device as claimed in claim 1, wherein the reflector is a reflector that can be switched between a reflecting position and a passing position, allowing, in the reflecting position, the reflection of a wave being propagated in the optical fiber at the first wavelength for the polarimetric characterization of the optical fiber and, in the passing position, the reflection of the wave by the sample for the polarimetric characterization of the assembly comprising the optical fiber and the sample.

5. The device as claimed in any one of the preceding claims, wherein the single-mode optical fiber (30) is a polarization-maintaining optical fiber.

6. The device as claimed in claim 5, wherein the single-mode optical fiber comprises a first section of a polarization-maintaining single-mode optical fiber and a second section of the same polarization-maintaining single-mode optical fiber, the sections being of the same length and connected together such that the fast axis of the first section is aligned with the slow axis of the second section and vice versa.

7. The device as claimed in any one of the preceding claims, further comprising, on the distal side of the optical fiber, means for focusing (42) a wave at the first wavelength for the characterization of a spot zone of the sample.

8. The device as claimed in claim 7, further comprising, on the distal side of the optical fiber, scanning means (46) for the polarimetric characterization of a set of spot zones of the sample.

9. A method for remote polarimetric characterization of a sample (S) comprising:

   - the emission of an incident light wave at at least one first wavelength ($\lambda_E$) intended to be propagated in a single-mode optical fiber (30);
   - the polarimetric characterization of the optical fiber at the first wavelength, comprising:

     ∘ the generation of a given number of polarization states of the incident light wave, called probe states, by means of a polarization state generator (20) arranged on the proximal side of the optical fiber;
     ∘ the analysis, by means of a polarization state analyzer (50) arranged on the proximal side of the optical fiber, for each probe state of the incident wave, of the polarization of the light wave obtained after propagation of the incident wave in the optical fiber (30), reflection by means of a reflector (40, 44) arranged on the distal side of the optical fiber and back propagation in the optical fiber (30);
     ∘ the determination of a Mueller matrix ($\mathbf{M_F}(\lambda_E)$) associated with the optical fiber at the first wavelength;

   - the polarimetric characterization of the assembly comprising the optical fiber and the sample at the first wavelength, comprising:

> ◦ by means of said polarization state generator (20) and polarization state analyzer (50), the analysis, for each probe state of the polarization, of a wave returned from the distal side of the fiber by the sample and propagated in the reverse direction in the optical fiber (30);
> ◦ the determination of a Mueller matrix ($M_T$) associated with said assembly at the first wavelength;

- the determination of the Mueller matrix ($M_O$) associated with the sample at the first wavelength from the Mueller matrices associated respectively with the optical fiber and with the assembly comprising the optical fiber and the sample.

10. The polarimetric characterization method as claimed in claim 9, comprising:

- the emission of a light wave at a second wavelength ($\lambda_{F1}$) distinct from the first wavelength ($\lambda_E$),

and wherein:

- the reflector is a spectral reflector allowing the reflection of a wave being propagated in the optical fiber at the second wavelength ($\lambda_{F1}$) for the polarimetric characterization of the optical fiber at the second wavelength ($\lambda_{F1}$) and the passage of the wave at the first wavelength ($\lambda_E$) for the polarimetric characterization of the assembly comprising the optical fiber and the sample at the first wavelength ($\lambda_E$);
- the determination of the Mueller matrix ($M_F(\lambda_E)$) associated with the optical fiber at the first wavelength comprises:

> ◦ from the polarimetric characterization of the optical fiber at the second wavelength ($\lambda_{F1}$), a Mueller matrix ($M_F(\lambda_{F1})$) associated with the optical fiber at the second wavelength ($\lambda_{F1}$);
> ◦ from the Mueller matrix associated with the optical fiber at the second wavelength ($\lambda_{F1}$), a Mueller matrix ($M_F(\lambda_E)$) associated with the optical fiber at the first wavelength ($\lambda_E$).

11. The polarimetric characterization method as claimed in claim 10, comprising:

- the emission of a wave at a third wavelength ($\lambda_{F2}$) distinct from the first and second wavelengths,

and wherein:

- the spectral reflector allows the reflection of waves being propagated in the optical fiber at the second and third wavelengths ($\lambda_{F1}$, $\lambda_{F2}$) for the polarimetric characterization of the optical fiber at the second and third wavelengths ($\lambda_{F1}$, $\lambda_{F2}$);
- the determination of the Mueller matrix ($M_F(\lambda_E)$) associated with the optical fiber at the first wavelength comprises:

> ◦ from a polarimetric characterization of the optical fiber at the second wavelength ($\lambda_{F1}$) and from a polarimetric characterization of the optical fiber at the third wavelength ($\lambda_{F2}$), respectively a Mueller matrix ($M_F(\lambda_{F1})$) associated with the optical fiber at the second wavelength ($\lambda_{F1}$) and a Mueller matrix ($M_F(\lambda_{F2})$) associated with the optical fiber at the third wavelength ($\lambda_{F2}$);
> ◦ from the Mueller matrices associated with the optical fiber at the second wavelength ($\lambda_{F1}$) and third wavelength ($\lambda_{F2}$), the Mueller matrix ($M_F(\lambda_E)$) associated with the optical fiber at the first wavelength ($\lambda_E$).

12. The polarimetric characterization method as claimed in claim 9, wherein the reflector is a reflector that can be switched between a reflecting position and a passing position, allowing, in the reflecting position, the reflection of a wave being propagated in the optical fiber at the first wavelength for the polarimetric characterization of the optical fiber and, in the passing position, the reflection of the wave by the sample for the polarimetric characterization of the assembly comprising the optical fiber and the sample.

13. The method as claimed in any one of claims 9 to 12, further comprising, on the distal side of the optical fiber, the focusing of a light wave at the first wavelength by the focusing means (42) for the characterization of a spot zone of the sample.

**14.** The method as claimed in claim 13, further comprising, on the distal side of the optical fiber, the scanning by scanning means (46) of the focused light wave for the polarimetric characterization of a set of spot zones of the sample.

ART ANTERIEUR

FIG.1A

ART ANTERIEUR

FIG.1B

| H | V | P | M | L | R | A |
|---|---|---|---|---|---|---|
| $\begin{bmatrix} 1 \\ 1 \\ 0 \\ 0 \end{bmatrix}$ | $\begin{bmatrix} 1 \\ -1 \\ 0 \\ 0 \end{bmatrix}$ | $\begin{bmatrix} 1 \\ 0 \\ 1 \\ 0 \end{bmatrix}$ | $\begin{bmatrix} 1 \\ 0 \\ -1 \\ 0 \end{bmatrix}$ | $\begin{bmatrix} 1 \\ 0 \\ 0 \\ 1 \end{bmatrix}$ | $\begin{bmatrix} 1 \\ 0 \\ 0 \\ -1 \end{bmatrix}$ | $\begin{bmatrix} 1 \\ \cos 2\theta \cos 2\varepsilon \\ \sin 2\theta \cos 2\varepsilon \\ \sin 2\varepsilon \end{bmatrix}$ |

ART ANTERIEUR

FIG.1C

EP 3 120 134 B1

ART ANTERIEUR

FIG.2

FIG.3

FIG.4

PARTIE DISTALE

PARTIE PROXIMALE

ON

OFF

S

S

200

$\lambda_E$

42

44

42

44

30

24

22

50

65

PSG

PSA

20

62

$D_E$

$\lambda_E$

12

70

FIG.5

FIG.6

EP 3 120 134 B1

FIG.7

FIG.8

FIG.9A

Angle d'incidence du faisceau sur la lame de verre (degrés)

FIG.9B

FIG.10A

FIG.10B

FIG.11A

Retard de phase linéaire induit par le compensateur (degrés)

FIG.11B

EP 3 120 134 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2007003840 A
- FR 2941047
- FR 2977033, d'Alouni
- EP 1411333 A

- US 6177995 B1
- US 6175412 B1
- US 7298480 B2

**Littérature non-brevet citée dans la description**

- **S. LU et al.** Interprétation of Mueller matrices based on polar décomposition. *J. Opt.Soc. Am. A,* 1996, vol. 13, 1106-1113
- **A. PIERANGELO et al.** *Opt. Express,* 2011, vol. 19, 1582-1593
- **A. PIERANGELO et al.** *J. Biomed. Opt.,* 2013, vol. 18, 046014
- **A. PIERANGELO et al.** *Opt. Express,* 2013, vol. 21, 14120-30
- **T.C. WOOD et al.** Polarization response measurement and simulation of rigid endoscopes. *Biomedical Optics express 463,* 2010, vol. 1

- **QI et al.** Narrow band 3X3 Mueller polarimetric endoscopy. *Biomedical Optics Express,* 2013, vol. 4
- **E. COMPAIN et al.** Complete Mueller matrix measurement with a single high frequency modulation. *Thin Solid Films,* 1998, 313-314, 47-52
- **E. COMPAIN et al.** Complete high-frequency measurement of Mueller matrices based on a new coupled-phase modulator. *Rev. Sci. Instrum.,* 1997, vol. 68, 2671-2680